# FASCICULE DE BREVET EUROPEEN

(11) **EP 2 219 477 B1**
(45) Date de publication et mention de la délivrance du brevet: **30.11.2011**
(21) Numéro de dépôt: 08840654.1
(22) Date de dépôt: 16.10.2008
(51) Int. Cl.: A23L 1/30, A23L 1/302, A23L 1/304, A61P 9/00, A61K 31/07, A61K 31/201, A61K 31/355, A61K 31/375, A61K 31/455, A61K 31/51, A61K 31/519, A61K 33/06, A61K 33/26, A61K 33/30, A61K 36/03

(54) **COMPOSITION DESTINEE A LA REGULATION DU METABOLISME DES LIPIDES**
ZUSAMMENSETZUNG FÜR DIE REGULATION DES LIPIDSTOFFWECHSELS
COMPOSITION FOR REGULATING LIPID METABOLISM

(30) Priorité: 16.10.2007 EP 07118598
(43) Date de publication de la demande: 25.08.2010
(73) Titulaire: EXICHOL SA, 1870 Monthey (CH)
(72) Inventeur: BOURGEOIS-LUGAND, Marie, Françoise, Yvonne, F-29530 Loqueffret (FR); WAHLI, Walter, CH-1112 Echichens (CH); EL KOCHAIRI, Ilhem, CH-1004 Lausanne (CH); PRADERVAND, Sylvain, CH-1022 Chavannes-Renens (CH); PARISOT, Gilles, Didier, CH - 1867 Ollon (CH)
(74) Mandataire: Jelsch, Emmanuel Edwin
(86) Numéro de dépôt international: PCT/IB2008/002815
(87) Numéro de publication internationale: WO 2009/050580

(56) Documents cités:
- WO-A-00/09141
- WO-A-99/48386
- WO-A-2005/095427
- JP-A- 62 061 925
- JP-A- 2001 010 947
- JP-A- 2002 275 088
- KINSELLA J E: "Grapeseed Oil: A rich source of linoleic acid" FOOD TECHNOLOGY, INSTITUTE OF FOOD TECHNOLOGISTS, CHICAGO, IL, US, vol. 28, no. 5, 1 janvier 1974 (1974-01-01), pages 58-60, XP009099089 ISSN: 0015-6639
- DATABASE WPI Week 199610 Thomson Scientific, London, GB; AN 1996-091609 XP002256701 -& JP 08 000219 A (NIPPON SYNTHETIC CHEM IND CO) 9 janvier 1996 (1996-01-09)
- DATABASE FSTA [Online] INTERNATIONAL FOOD INFORMATION SERVICE (IFIS), FRANKFURT-MAIN, DE; MIE-YOUN CHOI ET AL: "The effects of hot water soluble polysaccharides from Lentinus edodes on lipid metabolism in the rats fed yellow butter" XP002516137 Database accession no. 2000-00-j1596 & MIE-YOUN CHOI ET AL: "The effects of hot water soluble polysaccharides from Lentinus edodes on lipid metabolism in the rats fed yellow butter" JOURNAL OF THE KOREAN SOCIETY OF FOOD SCIENCE AND NUTRITION, vol. 29, no. 2, 2000, pages 294-299, DEP. OF FOOD SCI. & NUTR., PUSAN NAT. UNIV., PUSAN 609-735, KOREA
- DATABASE BIOSIS [Online] BIOSCIENCES INFORMATION SERVICE, PHILADELPHIA, PA, US; mai 2006 (2006-05), MORIHARA NAOAKI ET AL: "Aged garlic extract ameliorates physical fatigue" XP002516138 Database accession no. PREV200600462589 & BIOLOGICAL & PHARMACEUTICAL BULLETIN, vol. 29, no. 5, mai 2006 (2006-05), pages 962-966, ISSN: 0918-6158

## Description

### Domaine de l'invention

La présente invention concerne une composition destinée à la régulation du métabolisme des lipides et des méthodes utilisables dans le domaine de l'alimentation ainsi que le domaine nutraceutique et thérapeutique. Elle concerne notamment des additifs ou compléments alimentaires, une composition les contenant et leurs utilisations, notamment pour revitaliser le métabolisme de sujets, notamment humains.

### Discussion de l'état de la technique

La nutrition joue un rôle essentiel dans le maintien de la bonne santé ou du bon état général des sujets. En particulier, la nutrition permet de renforcer l'état général d'un sujet, de réduire la fatigue, d'améliorer la mémoire, de dynamiser certaines fonctions nécessaires à la vitalité de l'organisme, notamment de tonifier le métabolisme général et le métabolisme énergétique (lipides, sucres, protéines).

Le FENOFIBRATE® (EP0295637 WARNER LAMBERT) est utilisé pour réduire le taux de cholestérol et de triglycérides dans le sang, en association avec un régime. Il agit en activant le Peroxisome Proliferator Activated Receptor de type alfa (PPAR alpha). Cette activation entraîne une augmentation de la lipolyse et l'élimination du plasma des particules athérogènes riches en triglycérides (LDL et VLDL). Tout ceci aboutit à la réduction des taux de cholestérol et de triglycérides sanguins. On observe avec le FENOFIBRATE® les manifestations secondaires suivantes : troubles digestifs, douleurs musculaires, élévation des transaminases sanguines, céphalées (maux de tête), diarrhée et allergie cutanée. FENOFIBRATE® est contre-indiqué en cas d'insuffisance hépatique ou rénale grave ou en cas d'allergie

Les phytostérols sont des composés naturels présents dans les plantes, les oléagineux et les huiles qui en sont issues ainsi que dans l'huile du bois de pin. Les phytostérols utilisés pour la réduction du taux de cholestérol sont généralement tirés d'huiles végétales (soya, maïs, tournesol, colza) et se présentent sous la forme d'une substance cireuse peu soluble. Pour qu'ils s'intègrent bien aux aliments (margarines, sauces à salade, etc.) et qu'ils soient mieux absorbés, on les combine à des acides gras. En raison de leur structure chimique proche, les phytostérols entravent l'absorption du cholestérol en occupant ses sites d'absorption dans l'intestin. Bien que la description chimique des phytostérols remonte à 1922, ce n'est qu'au cours des années 1970 qu'on commence à évoquer sérieusement leur action bénéfique sur les taux de cholestérol. Des essais cliniques menés depuis le milieu des années 1970 démontrent que les phytostérols permettent de faire baisser de 8 % à 12 % le taux de cholestérol LDL (Low Density Lipoprotein Cholestérol « aussi appelé mauvais cholestérol »). La consommation d'aliments enrichis en phytostérols ou de suppléments de phytostérols peut réduire le taux sanguin de caroténoïdes (βcaroténoïdes, lycopène). Cet effet est attribuable à la diminution de l'absorption intestinale de ces substances. Les phytostérols sont contre-indiqués en cas de sitostérolémie et de certaines xanthomatoses. Il a été prouvé que les phytostérols sont inefficaces sur les triglycérides et que leur efficacité sur le LDL/cholestérol est malgré tout limitée.

Il est connu que les nutriments du genre oligoéléments, plantes, principes alimentaires (acides aminés) ou vitamines peuvent activer ou inhiber certaines fonctions de l'organisme ou avoir un effet neutre purement énergétique. La micronutrition consistant à apporter à l'organisme un ou plusieurs nutriments en quantités réduites a permis de résoudre partiellement les problèmes posés par la nutrition pondérale classique. Ces quantités réduites permettent aux nutriments d'être directement assimilables par le destinataire final qu'est la cellule, ce que ne permet pas la nutrition pondérale. Mais surtout la micronutrition a permis de résoudre le problème de la saturation des sites d'absorption. Ainsi, la micronutrition ne sature pas les sites d'absorption intestinale.

DE 19930221 A1 (MARCINOWSKI PETER) (2001-01-11) divulgue un aliment d'une valeur nutritionnelle améliorée, comprenant des levures, des vitamines, des huiles végétales, en particulier l'huile de lin, des algues marines et un mélange de minéraux. Les levures comprennent des traces de métaux et des minéraux, ainsi que des vitamines B- complexe.

Le document DATABASE WPI Week 200316 Derwent Publications Ltd., London; AN 2003-160118 XP002477227 & JP 2002 291419 A (FANKERU KK) (2002-10-08) divulgue une composition sous forme de capsule facile à préparer, comprenant une huile, vitamine B2, thiamine, vitamine B6, vitamine B12, l'acide folique, a biotine, l'acide pantothénique, les vitamines P, D, E, F et K, des caroténoïdes, de la levure contenant des métaux, l'acide ascorbique et la nicotinamide. L'huile utilisée peut être sélectionnée de l'huile de perilla, l'huile d'arachide, l'huile de germes de blé, l'huile d'olives, l'huile de pépins de raisin, l'huile de carthame, DHA, EPA, l'huile d'onagre.

RU-C1-2 159 564 (ORLOVA RAISA PETROVNA ET AL) (2000-11-27) divulgue une composition qui augmente la résistance vis-à-vis de l'irradiation et possède des propriétés stimulatrices, comprenant de la levure, des huiles végétales, et des substances actives, étant le kale marin, des noix et ou/du miel. Les levures comprennent des traces de métaux et des minéraux, ainsi que des vitamines B-complexe.

Le document DATABASE FSTA [Online] INTERNATIONAL FOOD INFORMATION SERVICE (IFIS), FRANKFURT-MAIN, DE; KINSELLA J E: "Grapeseed oil: a rich source of linoleic acid" XP002477226 Database accession no. 74-4-07-n0351 divulgue que l'huile de pépins de raisin peut être utilisée en tant qu'ingrédient fonctionnel dans des aliments pour réduire le taux sanguin de cholestérol.

WO 00/09141 A (WAKUNAGA OF AMERICA CO LTD ET AL) (2000-02-24) divulgue une composition pour réduire le taux sanguin d'homocystéine, comprenant un extrait d'ail, les vitamines B6 et B12 et de l'acide folique. Ainsi, le risque des maladies cardio-vasculaires comme les infarctus du myocarde est réduit. Il est spécifiquement divulgué que les préparations à base d'ail réduisent le taux sanguin de cholestérol.

US 2002/172729 A1 (KENTON KALEVI JOHN ET AL) (2002-11-21) décrit une composition pharmaceutique comprenant de l'acide ascorbique, de la vitamine E, du magnésium, des acides aminés, des flavonoïdes et du lycopène comme actifs, pour la prévention.des maladies cardio-vasculaires telle que les plaques d'athérome, des infarctus du myocarde. La poudre d'ail peut être présente.

WO 2005/095427 A (TAKARA BIO INC ET AL) (2005-10-13) divulgue une composition comprenant un polysaccharide sulfate dérivé d'algues du type Fucus d'un poids moléculaire réduit, pour la prévention ou le traitement des thromboses.

DATABASE WPI Week 199610 Derwent Publications Ltd., London; AN 1996-091609 XP002256701 -& JP 08 000219 A (NIPPON SYNTHETIC CHEM IND CO) (1996- 0 1-09) décrit une composition pour la prévention de la formation de cholestérol et des thromboses cérébrales, comprenant comme actifs un polysaccharide naturel tel que le carraghénane et de l'huile de poisson, telle que l'huile de thon ou de sardine.

JP 2002 275088 A (NAGAOKA HITOSHI) (2002-09-25) divulgue une composition pour l'inhibition de l'artériosclérose, comprenant un extrait du mycélium du shiitake (Cortinellus shiitake fungus).

WO 99/48386 A (STUECKLER FRANZ) (1999-09-30) divulgue une composition alimentaire basée sur des substances naturelles, ayant une activité préventive vis-à-vis des maladies cardio-vasculaires. Elle comprend de la lécithine, un extrait de vigne rouge et de l'acétate de tocophérol, ainsi que de l'huile de saumon, un extrait de shiitake, des vitamines B-complexe (acide folique, vitamines B1, B2, B6, B12, nicotinamide, pantothénol, biotine), un extrait d'ail, de l'acide ascorbique. La matrice de la composition peut être de la levure médicinale ou de l'huile d'olive. Il est divulgué que (1) l'huile d'olive réduit le taux sanguin de cholestérol à cause de sa teneur élevée en acide oléique; (2) les extraits d'ail ont un effet protecteur contre l'artériosclérose et la formation de plaques d'athérome; (3) les vitamines B-complexe réduisent le taux sanguin d'homocystéine et ainsi l'artériosclérose; (4) les extraits de shiitake réduisent la formation de plaques d'athérome, réduit le taux sanguin de cholestérol et le risque des thromboses; (5) les huiles de poisson réduisent le taux sanguin des triglycérides et l'agrégation des thrombocytes.

Malgré les solutions proposées par l'art antérieur, il n'existe cependant pas de composition efficace, ne présentant pas d'effet indésirable ou secondaire pour l'organisme et qui permette d'agir à la fois sur:
- la baisse des lipides circulants
- la prévention de la plaque d'athérome
- la prévention de la stéatose hépatique
- le contrôle du poids ; prévention de l'obésité en stabilisant la masse graisseuse corporelle, avec limitation de la taille des adipocytes
- l'augmentation du catabolisme oxydatif des lipides et de la consommation d'oxygène
- et l'amélioration de la performance physique et de l'endurance

### Brève description de l'invention

La présente invention, se propose de répondre à cette demande par l'élaboration d'une composition innovante destinée à la régulation du métabolisme des lipides chez l'homme et l'animal. Entre autre, la composition selon l'invention permet d'agir de manière significative sur le métabolisme des graisses et fournit des résultats particulièrement avantageux portant à la fois sur la baisse du cholestérol total (pouvant atteindre -18%), des LDL-C (pouvant atteindre -20%), et des triglycérides (pouvant atteindre -35%).

La présente invention concerne plus particulièrement une composition destinée à la régulation du métabolisme des lipides chez l'homme et l'animal. Cette composition comprend la combinaison de:
- 7µg à 700µg (par 100g/100ml) d'au moins deux huiles végétales sélectionnées parmi l'huile de colza, l'huile d'olive, l'huile de pépins de raisin et l'huile d'onagre,
- 10µg à 1000µg (par 100g/100ml) de minéraux chargés positivement choisis parmi le sodium, le magnésium et le calcium,
- 10µg à 1000µg (par 100g/100ml) de métaux choisis parmi le zinc et le fer,
- 7µg à 700µg (par 100g/100ml) de levures ou extraits de levure provenant du genre *Saccharomyces cerevisiae*, caractérisé en ce que ces dites levures ou extraits de levure sont enrichies en Sélénium,
- 7µg à 700µg (par 100g/100ml) de champignons ou d'extraits de champignon Shiitaké (mycelium)
- 6µg à 600µg (par 100g/100ml) d'au moins deux extraits végétaux de plante choisis parmi la criste marine, l'ail et la Vigne,
- 8µg à 800µg (par 100g/100ml) d'au moins une vitamine choisie parmi les vitamines A, B1, B9, C, E, F et PP
- 7µg à 700µg (par 100g/100ml) d'huile animale et de Coprah (Cocos nucifera)
- 6µg à 600µg (par 100g/100ml) d'au moins une algue choisie parmi Palmaria palmata (Dulse), Chondrus crispus (Carragaeen) et Fucus vesiculosus (Varech vésiculeux),
ainsi qu'un excipient pharmaceutiquement et/ou alimentairement autorisé.
Ledit excipient complètera avantageusement le volume de manière à obtenir les 100 ml de la composition selon l'invention.

Il a été démontré dans les exemples ci-après que la composition selon l'invention produit des effets synergiques inattendus et de loin supérieurs à ceux des ingrédients pris isolément qui ne permettent pas d'obtenir les résultats surprenants liés à la composition telle que décrite.

L'invention concerne également l'utilisation d'une composition telle que définie pour la préparation d'un additif alimentaire.

L'utilisation de la dite composition pour la préparation d'un médicament ou d'un produit nutritionnel destiné à la régulation du métabolisme des lipides chez l'homme et l'animal est également un des objets de la présente invention.

En particulier, la régulation du métabolisme des lipides consiste en l'entretien et/ou la régénération de l'organisme humain ou animal par le rééquilibrage et la redynamisation des fonctions générales dudit métabolisme comprenant :
la stimulation de la consommation des lipides par l'organisme et/ou
la diminution du taux plasmatique du cholestérol et/ou des triglycérides,

Le médicament ou le produit nutritionnel selon l'invention peut être utilisé pour traiter ou prévenir le syndrome métabolique, la formation de plaques d'athérome, la stéatose hépatique et/ou les maladies cardio-vasculaires.

D'autres avantages inattendus de la composition selon l'invention apparaîtront à la lecture de la description détaillée et des exemples de réalisation de l'invention.

### Brève description des figures

Figure 1 : Evolution des taux de LDL plasmatiques des souris ob/ob
Figure 2 : Evolution des taux de triglycérides plasmatiques des souris ob/ob
Figure 3 : Nombre de plaques aortiques d'athérosclérose chez les souris mâles APOE ko (à l'âge de 12 mois)
Figure 4 : Stéatose hépatique (foie fixé en paraffine, HE, grossissement 10x)
Figure 5 : Gain de poids des souris OB/OB mâles recevant une nourriture enrichie en graisse (semaine 4 vs semaine 0)
Figure 6a : Evolution du poids des souris mâles LDLr (ko)
Figure 6b : Evolution du poids des souris APOEko mâles (traités dès l'âge de 10 semaines)
Figure 6c : Pourcentage de la masse graisseuse des souris mâles LDLr (ko)
Figure 6d : Evolution de la masse graisseuse corporelle des souris mâles APOEko (traités dès l'âge de 10 semaines)
Figure 6e : Tissu adipeux blanc épididymal de souris mâles LDLr ko (fixation en paraffine, HE, grossissement 5x)
Figure 6f : Taille moyenne des adipocytes de souris mâles LDLr ko
Figure 7a : Courbe de consommation d'oxygène de souris mâles OB/OB (semaine 0)
Figure 7b : Courbe de consommation d'oxygène de souris mâles OB/OB (semaine 4)
Figure 7c : Différence d'aire sous la courbe (ASC) de consommation d'oxygène de souris mâles OB/OB
Figure 7d : Consommation d'oxygène à 3 mois des souris mâles APOEko (traités depuis l'âge de 10 semaines)
Figure 7e : Consommation d'oxygène à 6 mois des souris mâles APOEko (traités depuis l'âge de 10 semaines)
Figure 7f : Consommation d'oxygène à 9 mois des souris mâles APOEko (traités depuis l'âge de 10 semaines)
Figure 7g : Différence d'aire sous la courbe (ASC) de la consommation d'oxygène des souris mâles APOE ko (traités depuis l'âge de 10 semaines)
Figure 7h : Consommation cumulative d'oxygène par les souris mâles LDLr ko
Figure 8a : Production de chaleur à 3 mois des souris mâles APOEko (traités depuis l'âge de 10 semaines)
Figure 8b : Production de chaleur à 6 mois des souris mâles APOEko (traités depuis l'âge de 10 semaines)
Figure 8c : Production de chaleur à 9 mois des souris mâles APOEko (traités depuis l'âge de 10 semaines)
Figure 8d : Différence d'aire sous la courbe (ASC) de la production de chaleur des souris mâles APOE ko (traités depuis l'âge de 10 semaines)
Figure 8e : Production cumulative de chaleur par les souris mâles LDLr ko
Figure 9 : Densité des crêtes mitochondriales du muscle oxydatif de souris OB/OB après 4 semaines de traitement par la composition selon l'invention versus contrôle.
Figure 10 : Test de natation forcée de souris OB/OB (avec une charge de 7,5% du poids corporel)
Figure 11 a : Evolution des taux de triglycérides plasmatiques des souris mâles LDLr ko
Figure 11b : Evolution des taux de triglycérides plasmatiques des souris mâles APOE ko (traités depuis l'âge de 10 semaines)
Figure 12a : Taux de triglycérides plasmatiques à jeun des souris mâles LDLr ko (10 mois de traitement)
Figure 12b : Taux plasmatiques de triglycérides en post prandial de souris mâles LDLr ko (10 mois de traitement)
Figure 13a : Evolution du poids corporel des souris mâles PPARa(ko)LDLr(ko)
Figure 13b : Taux de triglycérides plasmatiques NON à jeun des souris mâles LDLrkoPPARako après 10 mois de traitement
Figure 13c : Consommation cumulative d'oxygène en 24h des souris mâles LDLr(ko)PPARalpha(ko)
Figure 13d : Production cumulative de chaleur en 24h des souris mâles LDLr(ko)PPARalpha(ko)
Figure 14 : Test de transactivation de PPARalpha par la composition selon l'invention dans des cellules NIH3T3 transfectées par 5UAS-LUC + Renilla + Gal4 ou Gal4-alphaLBD
Figure 15 : Gènes régulés par PPAR alpha et surexprimés dans le muscle de souris OB/OB après un mois de traitement
Figure 16 : Gènes surexprimés dans le muscle de souris OB/OB après un mois de traitement, impliqués dans la transmission neuro-musculaire, la trophicité, la synthèse protéique et la contractilité musculaires.
Figure 17A : Diagramme de Venn représentant le nombre de gènes affectés par le traitement de la composition selon l'invention dans le foie, le muscle squelettique et le tissu adipeux. Les comparaisons ont été effectuées entre les animaux contrôles et ceux traités par la composition selon l'invention (n=7 par groupe). Les gènes exprimés de façon différentielle ont une probabilité d'être un faux positif inférieure à 10%.

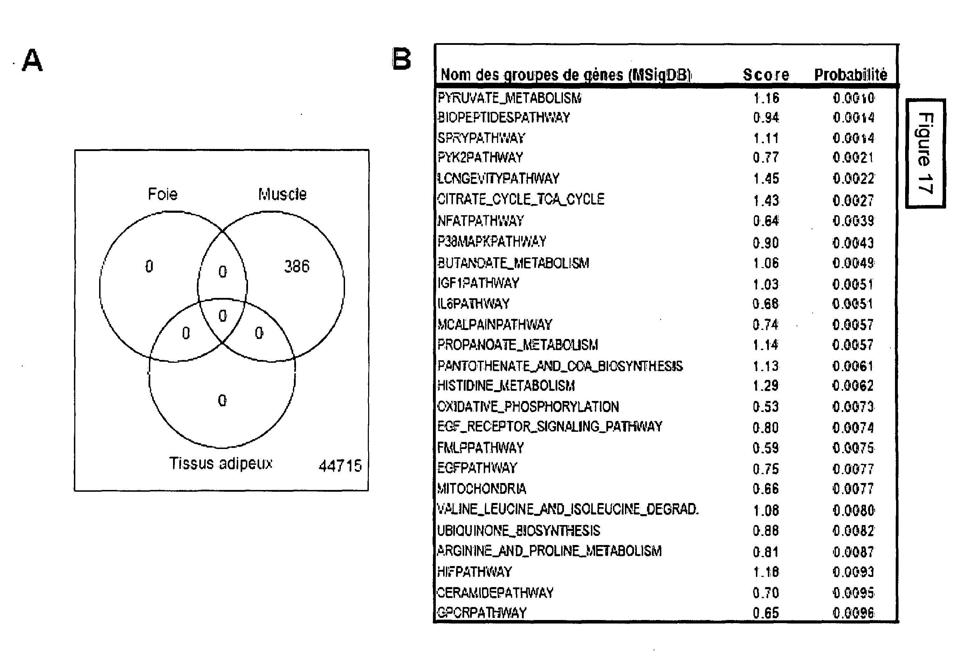

Figure 17B : Analyse par groupe de gènes des effets de la composition selon l'invention sur le muscle squelettique. Les groupes de gènes représentant les voies de signalisation et les voies métaboliques ont été compilés par des experts et sont disponibles dans la base de données « Molecular Signatures Database » (MSigDB). Les voies enrichies de façon significatives par la composition selon l'invention sont indiquées sur le tableau (P < 0.01, taux de faux positifs < 12%). Le nom du groupe de gènes tel qu'il est nommé dans MSigDB, le score d'enrichissement (positif = enrichi, négatif = appauvri), et la valeur de probabilité sont indiqués.

### Description détaillée de l'invention

La présente invention relate de la mise au point d'une composition utilisable en tant qu'additifs ou compléments alimentaires ou nutritifs et/ou nutraceutique et/ou thérapeutique, ayant des propriétés particulièrement avantageuses sur le métabolisme des lipides. Les compositions de l'invention sont utilisables dans le domaine de la nutrition, chez l'homme ou l'animal, y compris à titre préventif.

De manière surprenante, la composition selon l'invention permet de provoquer chez l'homme une diminution simultanée et substantielle du cholestérol total (pouvant atteindre -18%) et des triglycérides (pouvant atteindre -35%), et une diminution moyenne des taux de LDL (pouvant atteindre -20%). La composition de l'invention présente ainsi l'avantage d'agir à la fois sur le cholestérol et sur les triglycérides, et ceci sans effet secondaire identifié. Ces propriétés confèrent à la composition de l'invention des applications particulièrement avantageuses dans le domaine de la revitalisation du métabolisme des lipides, par exemple chez des sujets atteints ou susceptibles d'être atteints de syndrome métabolique ou d'autres désordres liés à une dysrégulation du métabolisme des lipides.

Par ailleurs, les études effectuées montrent que les effets de la composition de l'invention résultent d'un mécanisme d'action avantageux et différent des médicaments ou préparations existantes. En effet, l'action sur la régulation du métabolisme des lipides découle d'une action positive (stimulante ou revitalisante) sur la consommation du cholestérol, plutôt que d'une action inhibitrice sur la synthèse ou l'adsorption du cholestérol. Cette action sur la consommation du cholestérol s'exerce notamment au niveau du muscle squelettique.

De plus, les données obtenues montrent que l'action de la composition de l'invention mobilise moins de voies métaboliques ou génétiques que celle de médicaments disponibles tels que les fibrates. Ainsi, elle est obtenue par une modulation de l'expression d'un nombre restreint de gènes, en comparaison des médicaments utilisés à ce jour.

La préparation de l'invention présente une composition particulière et une action biologique importante, médiée par un mécanisme particulièrement avantageux.

Un premier objet de la présente invention réside dans une composition destinée à la régulation du métabolisme des lipides chez l'homme et l'animal. Cette composition comprend la combinaison de:
- 7µg à 700µg (par 100g/100ml) d'au moins deux huiles végétales sélectionnées parmi l'huile de colza, l'huile d'olive, l'huile de pépins de raisin et l'huile d'onagre,
- 10µg à 1000µg (par 100g/100ml) de minéraux chargés positivement choisis parmi le sodium, le magnésium et le calcium,
- 10µg à 1000µg (par 100g/100ml) de métaux choisis parmi le zinc et le fer,
- 7µg à 700µg (par 100g/100ml) de levures ou extraits de levure provenant du genre *Saccharomyces cerevisiae,* caractérisé en ce que ces dites levures ou extraits de levure sont enrichies en Sélénium,
- 7µg à 700µg (par 100g/100ml) de champignons ou d'extraits de champignon Shiitaké (mycelium)
- 6µg à 600µg (par 100g/100ml) d'au moins deux extraits végétaux de plante choisis parmi la criste marine, l'ail et la Vigne,
- 8µg à 800µg (par 100g/100ml) d'au moins une vitamine choisie parmi les vitamines A, B1, B9, C, E, F et PP
- 7µg à 700µg (par 100g/100ml) d'huile animale et de Coprah (Cocos nucifera)
- 6µg à 600µg (par 100g/100ml) d'au moins une algue choisie parmi Palmaria palmata (Dulse), Chondrus crispus (Carragaeen) et Fucus vesiculosus (Varech vésiculeux),
ainsi qu'un excipient pharmaceutiquement et/ou alimentairement autorisé.

De manière préférée, la composition selon l'invention comprend de l'huile de poisson des mers froides (Oleum Pisci mare fresca) en tant qu'huile animale.

Selon un mode de réalisation préféré, la composition selon l'invention contient au moins deux vitamines choisies parmi les vitamines A, B1, B9, C, E, F et PP.

Plus particulièrement, la composition selon l'invention comprend préférablement de :
- 7µg à 700µg (par 100g/100ml) d'huile de colza, de l'huile d'olive, de l'huile de pépins de raisin(s), de l'huile d'onagre,
- 10µg à 1000µg (par 100g/100ml) de sodium, de magnésium et de calcium,
- 10µg à 1000µg (par 100g/100ml) de zinc et le fer,
- 7µg à 700µg (par 100g/100ml) de levures ou extraits de levure de *Saccharomyces cerevisiae,* enrichis en Sélénium,
- 7µg à 700µg (par 100g/100ml) de mycélium ou d'extrait de mycélium de Shiitake,
- 6µg à 600µg (par 100g/100ml) de criste marine, d'ail et de vigne,
- 8µg à 800µg (par 100g/100ml) de vitamines A, B1, B9, C, E, F et PP
- 7µg à 700µg (par 100g/100ml) d'huile de poisson des mers froides et de Coprah,
- 6µg à 600µg (par 100g/100ml) de Palmaria palmata (Dulse), Chondrus crispus (Carragaeen) et Fucus vesiculosus (Varech vésiculeux).

La composition de l'invention peut préférablement comprendre des excipients ou additifs, comme par exemple : eau, huile, lactose-saccharose ou lactose-amidon, fructo-oligosaccharides, sorbitol, phosphate dicalcique.

Parmi les excipients utilisés en alimentaire (en tant qu'additifs alimentaires) : colorants, conservateurs (sorbate de potassium, benzoate de sodium), arômes, anti-oxydants (caroténoïdes, vitamines C et E, flavonoïdes), émulsifiants (lécithine, mono et diglycérides d'acides gras), stabilisants et gélifiants (lécithine, lactate de potassium, agar agar, carraghénanes, alginate de sodium), exhausteurs de goût (sels de l'acide glutamique, inosinate de sodium), acidifiants (acide citrique, maltate de sodium), anti-agglomérants (stéarate de magnésium, dioxyde de silicium), édulcorants (sorbitol, sodium saccharinate).

La composition de l'invention peut se présenter sous différentes formes, et notamment en tant que complément ou additif alimentaire destinée à être incorporable dans tous types de support alimentaires et/ou boissons sous forme solide, liquide, de gel, filmstrips, pâte, poudre, gomme, etc. Elle peut être conditionnée dans tout type de support adapté, comme des flacons, boîtes, blisters, fioles, ampoules, etc. Elle est typiquement adaptée à une administration orale.

Par ailleurs, la composition de l'invention peut être utilisée en tant que complément ou additif alimentaire, sous forme mélangée à d'autres produits, des boissons, des condiments, etc. Par exemple, la composition selon l'invention peut être incorporée dans des supports alimentaires et/ou boissons, tels que les margarines, les huiles, les laits en poudre, les produits laitiers (crèmes dessert, yaourts lactés, etc.), les barres céréalières, les boissons (eaux minérales, jus de fruits, etc.), les sels, les condiments, les sauces, etc.

Les déposants se sont attachés à mettre au point une composition, utilisable comme actif alimentaire ou comme complément ou additif alimentaire, comme nutraceutique et/ou comme médicament, afin d'améliorer l'état de sujets. Plus particulièrement, les déposants ont recherché à mettre au point une composition dotée d'une action détoxicante et anti-oxydante, d'une action sur l'assimilation du cholestérol circulant et son élimination, sur l'augmentation du turn-over des lipides, d'une action stimulatrice sur le métabolisme basal régulant une partie de la combustion et de la vitalité des cellules et de l'organisme, et/ou d'une action sur la transformation bénéfique des acides gras poly-insaturés, par exemple leur effet anti-inflammatoire.

Ces recherches ont notamment été ciblées sur la mise au point d'une composition présentant des propriétés sur:
■ la fonction détoxication : stimulation et régulation des cellules hépatiques, de la vésicule biliaire et de l'intestin grêle. Cette fonction est la première action de prévention et de régulation métabolique lipidique. Elle permet l'évacuation des toxines, l'élimination des déchets, provenant de l'alimentation, de l'activité cellulaire, de la destruction de micro-organismes, de l'utilisation de médicaments, du métabolisme des graisses, de la pollution environnementale.
■ la défense anti-radicalaire agissant sur la diminution de l'oxydation des LDL et la neutralisation de dérivés actifs de l'oxygène
■ l'augmentation de la perméabilité des membranes cellulaires
■ le métabolisme basal régulant une partie de la combustion et de la vitalité cellulaire
■ la régulation du métabolisme des lipides

Ces recherches ont conduit à sélectionner différents types d'ingrédients, et à mettre au point et tester différentes combinaisons possibles de ces ingrédients, et à retenir une composition telle que définie ci-dessus, comprenant:
- des huiles végétales,
- des minéraux,
- des métaux (oligoéléments),
- des levures ou extraits de levure(s),
- de champignons ou extraits de champignons,
- des algues marines ou leurs extraits,
- des extraits végétaux (plantes), et
- des vitamines.

Les oligo-éléments sont les éléments du tableau de Mendeleiev, métaux ou métalloïdes que l'on retrouve dans l'organisme.

Les plantes ou extraits végétaux sont de façon générale les plantes ayant une action phytothérapique encore appelées simples. Ce sont des plantes ou végétaux pour lesquels les effets sont reconnus scientifiquement ou connus par tradition.

Les vitamines peuvent être toute substance ayant la fonction de vitamine pour l'organisme.

De manière préférée, les huiles végétales comprennent aux moins deux huiles choisies parmi l'huile de colza, l'huile d'olive, l'huile de pépins de raisin(s) et l'huile d'onagre. Avantageusement, la composition comprend au moins trois huiles végétales, plus préférentiellement elle comprend l'huile de colza, l'huile d'olive, l'huile de pépins de raisin(s) et l'huile d'onagre.

L'huile de colza (Oleum Brassica napus oleifera) est constituée de 98% de triesters d'acides gras ; les 2% restants sont riches en stérols et tocophérols (dont la vitamine E). C'est une huile riche en acide alpha-linolénique, acides gras poly-insaturés Oméga 3, en acides gras mono-insaturés Oméga 6 (avec un rapport intéressant Omega3:6 de 1:2,5) ; et seulement 6 à 8% d'acides gras saturés.

L'huile d'olive (Oleum Olea europea) est une huile riche en acide oléique : acide gras mono-insaturés (>75%), Oméga 6 (8%); l'huile d'olive contient des vitamines A, E et K. Le rapport vitamine E / AGPI (Acides Gras Poly Insaturés) est le plus élevé de toutes les huiles.

L'huile de pépins de raisin (Oleum Vitis vinifera) est une huile équilibrée en acide linoléique (alpha-linoléique et béta-linoléique), en acides oléique, palmitique et stéarique. Plus de 70% d'Omega 6. Fortement insaturée : le rapport poly-insaturés/saturés > 5.

L'huile d'onagre (Oleum Oenothera biennis) est une huile Oméga 6 équilibrée en acide linoléique, en acide gamma-linolénique, en acides oléique et stéarique.

Dans un mode de réalisation particulier, la composition comprend de l'huile de colza, de l'huile d'olive, de l'huile de pépins de raisin(s) et de l'huile d'onagre.

De manière préférée, les minéraux comprennent un ou plusieurs minéraux chargés positivement, choisis de préférence parmi le sodium, le magnésium et le calcium.

Le Sodium permet la régulation acide-base de l'organisme et du métabolisme cellulaire. Il joue un rôle déterminant dans la dépolarisation cellulaire qui est à l'origine de l'excitabilité et de la conduction des influx (en particulier neuromusculaire et cardiaque), dans le maintien de l'équilibre acido-basique, de la pression osmotique, dans l'équilibre des échanges liquidiens et ioniques de l'organisme.

Le Magnésium est indispensable à l'équilibre des canaux ioniques. Il agit comme cofacteur enzymatique et module les systèmes de transport du NA+ et du K+ dans tous les tissus ; il est le régulateur physiologique du Calcium dans l'équilibre des échanges cellulaires. Le Magnésium joue un rôle de stabilisateur des différents organites intracellulaires : il stabilise les ribosomes qui produisent les protéines, maintient la production d'énergie par les mitochondries car indispensable à la synthèse des molécules d'A.T.P. Cette production d'énergie est la base de tous les mécanismes de vie cellulaire et de la vitalité globale de l'organisme. Le Magnésium est indispensable à la synthèse des protéines fondamentales à la construction cellulaire (certains acides aminés, ADN et ARN).
Le Calcium est impliqué dans de nombreuses réactions enzymatiques. Il permet la transmission des informations au niveau cellulaire en tant que second messager ce qui induit la transmission de l'influx nerveux, la contraction musculaire (par la formation d'actinomyosine), la stimulation des cellules sécrétrices (hormones comme l'insuline) et la libération des neuromédiateurs.

Dans un mode de réalisation particulier, la composition comprend du sodium, du magnésium et du calcium.

De manière préférée, les métaux comprennent un ou plusieurs métaux choisis parmi le zinc et le fer.

Le Zinc intervient dans l'activité de près de 200 enzymes (en particulier dans des systèmes enzymatiques comme les oxydoréductases, l'alcool déshydrogénase, la cytochrome réductase et la SOD ou Superoxydismutase). Les enzymes concernés par le Zinc ont une importance métabolique considérable: la glycolyse, la voie des pentoses, la néoglucogénèse, le métabolisme des lipides, et des acides gras.
Le Zinc est un métal activateur de la plupart des coenzymes nécessaires au métabolisme énergétique.
Le Zinc joue un rôle très important dans l'équilibre acido-basique (anhydrase carbonique), dans l'inflammation, dans la différenciation cellulaire, dans la défense endogène anti-radicalaire.

Le Zinc est un cofacteur hormonal (hormone de croissance, thyroïde, cortico-surrénales) et est indispensable à la transcription de la chaîne d'ADN (ARN-polymérase).
Le Zinc stabilise les membranes cellulaires en se couplant avec les groupements thiols en leur évitant ainsi de réagir avec le fer évitant ainsi une production de H2O2 radical libre très instable. Il intervient particulièrement dans le métabolisme de la vitamine A (mobilisation au niveau du foie, formation du rétinol).
Il stabilise les structures protéiques et joue un rôle dans l'expression des gênes.

Composant des cytochromes, le fer est indispensable à la détoxication et à la fabrication des hormones thyroïdiennes. Il fait partie de sites actifs de protéines (dites " protéines à fer ") qui possèdent un rôle majeur dans l'organisme : l'hémoglobine, la myoglobine, et les cytochromes.

Dans un mode de réalisation particulier, la composition comprend du zinc et du fer.

De manière préférée, les levures ou extraits de levure sont des levures du genre Saccharomyces, ou des extraits de telles levures (par exemple des préparations membranaires, vésiculaires, protéiques, etc...). Il s'agit tout particulièrement de levures (ou extraits) du genre Saccharomyces cerevisiae. Dans un mode particulier de réalisation, on utilise des (extraits de) levures enrichies en Sélénium, qui possède des propriétés anti-oxydantes.

D'autres levures du genre Saccharomyces peuvent également être utilisées comme par exemple celles qui sont utilisées dans :
- l'industrie agro-alimentaire : saccharomyces boulardii
- la fermentation alcoolique :
   - saccharomyces cerevisiae (« fermentation haute » pour le vin, la bière)
   - saccharomyces uvarum (« fermentation basse » pour des bières type Lager)
   - schizosaccharomyces pombe (bière d'Afrique)
   - aspergillus (saké)
- ou aussi torulaspora delbrueckii et candida stellata (initialement présentes dans le moût): augmentation des esters et réduction de la formation d'acidité volatile

De manière préférée, des extraits végétaux comprennent un ou plusieurs extraits de plante(s). Plus préférentiellement, la composition comprend, à titre d'extrait végétal, au moins deux extraits végétaux choisis parmi la criste marine, l'ail et la vigne. Avantageusement, la composition comprend la criste marine, l'ail et la vigne.

Le terme "extrait" végétal désigne au sens de la présente invention toute préparation obtenue à partir de tout ou partie du végétal considéré. Il peut s'agir d'un broyat, d'un filtrat, de graines, de feuilles, de tiges, d'écorce, etc., ou de combinaisons. L'extrait peut être préparé par des techniques classiques. Typiquement, l'extrait comprend des cellules végétales, qui peuvent être intactes ou non.

La Criste marine (Crithmum maritimum) est très riche en minéraux : zinc, fer, magnésium, cuivre et manganèse, en vitamines A, E, B1, B2. Elle présente une action détoxifiante.

L'ail (Allium sativum) est riche en vitamine C, en zinc, en manganèse, et possède une action hypocholestérolémiante. L'ail se caractérise par la présence de substances soufrées originales (trisulfure d'allyle, ajoène E) ayant des effets bénéfiques sur la fluidité sanguine (réduction de l'aggrégation plaquettaire) et le taux de cholestérol sanguin (diminution de la synthèse des triglycérides) : intérêt sur le plan cardio-vasculaire.

Le raisin ou vigne (Vitis vinifera) est très riche en vitamines A et B et en sels minéraux : manganèse, potassium, calcium. Le raisin draine la vésicule biliaire et le foie. Il est très riche en antiradicaux libres.

La composition selon l'invention comprend au moins une algue ou un extrait d'algue choisi parmi parmi Palmaria palmata (Dulse), Chondrus crispus (Carragaeen) et Fucus vesiculosus (Varech vésiculeux).

La Dulse (Palmaria palmata) est très riche en provitamine A, capitale dans le contrôle hormonal, et en vitamine C à action antiradicalaire. Riche en acides aminés essentiels.

Le varech vésiculeux (Fucus vesiculosus) est riche en fucostérol : stérol qui présente des propriétés hypocholestérolémiantes, comme le béta-sitostérol des végétaux. Il est également très riche en iode et en fer.

Le Carragaeen (Mousse d'Irlande - Chondrus Crispus) est riche en acides gras et équilibré entre les acides gras Oméga 3 et Oméga 6 ainsi qu'en acides gras insaturés permettant l'assimilation du cholestérol. Il est également riche en acides aminés et en Oligo-métaux (particulièrement Iode, Zinc et Fer); contient toutes les vitamines.

Dans un mode de réalisation particulier, la composition comprend des extraits de Palmaria palmata (Dulse), de Chondrus crispus (Carragaeen) et de Fucus vesiculosus (Varech vésiculeux).

La composition selon l'invention comprend au moins un champignon ou extrait de champignon Shiitaké (mycélium). Le mycélium de Shiitake (Lentinus edodes) est très riche en acides aminés, oligo-éléments et vitamines. Il présente des propriétés hypocholestérolémiantes et pro-immunitaires.

De manière préférée, les vitamines comprennent une ou plusieurs vitamines choisies parmi les vitamines A, B1, B9, C, E, F et PP. Dans un mode préféré, la composition comprend au moins deux vitamines différentes, plus préférentiellement au moins trois, quatre, cinq ou six vitamines différentes, choisies parmi les vitamines indiquées ci-dessus.

La vitamine A (rétinol) est estérifiée à l'intérieur de la cellule intestinale, incorporée aux chylomicrons, excrétée dans la lymphe et rejoint la circulation générale par le canal lymphatique. La vitamine A stabilise les membranes cellulaires, la biosynthèse et la régulation des hormones stéroïdes. La synthèse de certaines protéines est également sous la dépendance de la vitamine A.

La vitamine B1 (thiamine) fournit la cellule en acide NADPH2, qui a une importance primordiale dans la synthèse des lipides, stérols et acides gras. C'est un des chaînons indispensable de la néolipidogénèse.

La vitamine B9 (acide folique) joue un rôle important dans le métabolisme de la sérine qui est transformée en Acétyl coenzyme A.

La vitamine C (acide ascorbique) joue un rôle biochimique important aux stades initiaux du métabolisme des lipides, en association avec diverses hydroxylases. Les hydroxylases dépendantes du cytochrome P 450 microsomal catalysent en présence de l'acide ascorbique la transformation du cholestérol en acides biliaires.

La vitamine E (alpha-tocophérol) accompagne les chylomicrons dans les canaux lymphatiques jusqu'à la circulation générale. Dans le plasma l'alpha tocophérol est lié aux diverses catégories de lipoprotéines : aux LDL qui portent 40 à 60% des tocophérols et aux HDL qui en portent 35 %. Son taux est étroitement corrélé avec celui des lipides totaux et du cholestérol. Elle présente un effet antioxydant: tamponnement des radicaux libres. Elle participe à la formation et à la structure des phospholipides membranaires et possède une action stabilisatrice sur les membranes cellulaires.

La vitamine F (acide linoléique) est un acide gras insaturé indispensable pour la synthèse et la protection des lipides.

La vitamine PP (niacine) présente un effet hypocholestérolémiant (par stimulation de la protéine lipase ou par inhibition de la lipolyse médiée par l'AMP cyclique dans le tissu adipeux). C'est par ailleurs un acteur crucial dans le métabolisme énergétique cellulaire par son intervention dans tous les phénomènes d'oxydoréduction de l'organisme.

Dans un mode de réalisation particulier, la composition comprend de la vitamine A, de la vitamine B1, de la vitamine B9, de la vitamine C, de la vitamine E, de la vitamine F et de la vitamine PP.

Par ailleurs, dans un mode préféré de mise en oeuvre, la composition de l'invention comprend en outre une huile animale, notamment une huile de poisson, en particulier une huile de poisson des mers froides (Oleum pisci mare fresca). Cette huile est riche en acide gras de la série Oméga 3. Les acides gras Oméga 3 diminuent le taux sanguin des triglycérides en diminuant la synthèse hépatique des triglycérides, en diminuant les VLDL dans le sang et leur richesse en triglycérides, ce qui permet un métabolisme plus rapide. Les Oméga 3 permettent une bonne fluidité membranaire.

Par ailleurs, dans un mode particulièrement préféré de mise en oeuvre, la composition de l'invention comprend en outre (Cocos nucifera). Le Coprah est riche en acide gras et représente un régulateur intestinal.

Un objet particulier de l'invention concerne ainsi une composition comprenant de l'huile de colza, de l'huile d'olive, de l'huile de pépins de raisin(s), de l'huile d'onagre, une huile de poisson des mers froides, du Coprah, du sodium, du magnésium, du calcium, du zinc, du fer, un (extrait de) levure(s) Saccharomyces cerevisiae, de préférence enrichie en sélénium, des extraits végétaux de criste marine, d'ail, de Palmaria palmata (Dulse), de Chondrus crispus (Carragaeen), de Fucus vesiculosus (Varech vésiculeux), de Shiitake (mycelium) et de vigne, de la vitamine A, de la vitamine B1, de la vitamine B9, de la vitamine C, de la vitamine E, de la vitamine F et de la vitamine PP.

Selon les familles d'ingrédients, les quantités préférentielles sont déterminées pour :
- huiles végétales (l'huile de colza, de l'huile d'olive, de l'huile de pépins de raisin(s), de l'huile d'onagre) : 28µg à 280µg/100g ou 100ml
- oligoéléments : minéraux (sodium, magnésium, calcium) et métaux (zinc et fer) : 40µg à 400µg/100g ou 100ml
- levures ou extraits de levure(s) Saccharomyces cerevisiae, de préférence enrichie en sélénium : 28µg à 280µg/100g ou 100ml
- champignons ou extraits de champignons (Shiitake mycelium) : 28µg à 280µg/100g ou 100ml
- algues marines ou leurs extraits (Palmaria palmata (Dulse), Chondrus crispus (Carragaeen) et Fucus vesiculosus (Varech vésiculeux): 24µg à 240µg/100g ou 100ml
- huile de poisson des mers froides et Coprah : 28µg à 280µg/100g ou 100ml
- extraits végétaux de plantes (criste marine, d'ail et de vigne): 28µg à 280µg/100g ou 100ml
- vitamines (A, B1, B9, C, E, F et PP): 32µg à 320µg/100g ou 100ml.

La composition selon l'invention pourra être avantageusement préparée suivant par exemple le procédé de préparation décrit au point A de la partie expérimentale ou par toute autre méthodologie ou technique connue ou développée par l'homme du métier. L'invention peut être mise en oeuvre chez tout mammifère, en particulier chez les humains, adultes, âgés ou enfants. La composition de l'invention est sans effet secondaire connu et peut être administrée selon des régimes variés, dépendant du sujet. Elle peut être prise comme seul traitement ou en accompagnement d'un autre traitement, alimentaire ou médical.

Préférablement, la dose journalière de la composition à administrer, ingérer ou a appliquer à un individu est idéalement comprise entre est de 4 à 40µg. Bien entendu cette dose peut être soumise à des variations selon l'individu, son âge, sexe, état de santé etc.. Il appartient aux professionnels de la santé, aux diététiciens et autres spécialistes d'ajuster cette dose en fonction des paramètres individuels à prendre en compte.

L'invention concerne également l'utilisation d'une composition telle que définie pour la préparation d'un complément alimentaire ou nutritionnel ou également additif alimentaire.

Par « complément alimentaire ou nutritionnel » on entend suivant la Directive européenne 2002/46/CE du 10 juin 2002 (complétée par le Règlement CE 1925/2006 du 20 décembre 2006) les « denrées alimentaires dont le but est de compléter le régime alimentaire normal et qui constituent une source concentrée de nutriments ou d'autres substances ayant un effet nutritionnel ou physiologique, seuls ou combinés, commercialisés sous forme de dose, à savoir les formes de présentation telles que les gélules, les pastilles, les comprimés, les pilules et autres formes similaires, ainsi que les sachets en poudre, les ampoules de liquides, les flacons munis d'un compte-gouttes et les autres formes analogues de préparation liquide ou en poudre destinées à être prises en unités mesurées de faible quantité ».
Cela comprend notamment, mais non exclusivement, des vitamines et des minéraux, des acides aminés, des acides gras essentiels, des fibres, divers plantes et extraits végétaux.

Comme indiqué précédemment, la composition selon l'invention présente des propriétés avantageuses dans la régulation du métabolisme des lipides.
En particulier un objet de l'invention concerne une méthode pour réguler le métabolisme des lipides chez un sujet, comprenant l'administration, l'application ou l'ingestion d'une composition telle que définie dans la présente invention.

Ainsi la composition selon l'invention peut être utilisée pour la préparation d'un médicament ou d'un produit nutritionnel destiné à la régulation du métabolisme des lipides chez l'homme et l'animal.

En particulier, la régulation du métabolisme des lipides consiste en l'entretien et/ou la régénération de l'organisme humain ou animal par le rééquilibrage et la redynamisation des fonctions générales dudit métabolisme comprenant :
la stimulation de la consommation des lipides par l'organisme et/ou
la diminution du taux plasmatique du cholestérol et/ou des triglycérides,
et de ce fait, la composition selon l'invention favorise un retour aux normes de santé.

Le médicament ou le produit nutritionnel selon l'invention est également destiné au traitement ou à la prévention du syndrome métabolique, de la formation de plaques d'athérome, de la stéatose hépatique et/ou des maladies cardio-vasculaires. La composition de l'invention est particulièrement efficace pour revitaliser le métabolisme des lipides, et est donc particulièrement utile chez des patients atteints de syndrome métabolique ou présentant un risque de développer un syndrome métabolique. Le terme "syndrome métabolique" désigne un ensemble de perturbations métaboliques qui prédispose fortement à la progression des maladies cardio-vasculaires, dont l'athérosclérose et les accidents vasculaires cérébraux (AVC). Le syndrome métabolique désigne une série de problèmes liés à un mauvais métabolisme corporel et dont certaines perturbations sont caractérisées par une obésité abdominale, une élévation des triglycérides et/ou du cholestérol et/ou une hypertension artérielle.

Plus particulièrement et suivant un mode de réalisation préféré de l'invention, le syndrome métabolique comprend les maladies liées au contrôle du poids tel que l'obésité, l'amaigrissement ou la stabilisation de la masse graisseuse corporelle.

Un objet de la présente invention est également le fait de pouvoir stimuler la consommation des lipides par l'organisme en favorisant l'augmentation du métabolisme oxydatif et de la consommation d'oxygène. En particulier, la composition telle que définie dans la présente invention favorise une bonne utilisation des lipides par les muscles.

Un autre objet de la présente invention est l'amélioration de l'endurance chez un sujet par l'administration ou l'ingestion du médicament ou du produit nutritionnel selon l'invention. Il a été prouvé dans les exemples ci-dessous que le médicament ou le produit nutritionnel selon l'invention augmente sensiblement la motricité musculaire en améliorant la trophicité et la contractilité du muscle.

De manière surprenante, le médicament ou le produit nutritionnel selon l'invention participe de manière significative à la baisse du taux plasmatique du cholestérol et/ou des triglycérides (voir exemples).
Ainsi l'invention concerne l'utilisation de la composition telle que définie pour l'entretien, la régénération de l'organisme par le rééquilibrage et la redynamisation de la fonction générale du métabolisme des lipides.

Par ailleurs, le médicament ou le produit nutritionnel selon l'invention permet de diminuer de manière inattendue l'absorption intestinale des lipides alimentaires.

La composition suivant l'invention peut également servir pour la nutrition animale ou humaine.

Avantageusement la composition selon l'invention peut également être employée en cosmétique et en particulier dans les soins et la vitalisation de la peau.

Selon la destination de la composition, sa formulation définitive peut être ajustée par l'homme du métier, en suivant l'enseignement de la présente demande.

Ainsi, pour favoriser une action détoxicante, on privilégiera dans les compositions certains ingrédients d'origine végétale, comme l'ail et/ou la vigne, qui améliore la fonction vésiculaire ainsi que les champignons tels que mycélium de Shiitake, qui ont des propriétés de détoxication des cellules hépatiques, et également la vitamine B1, qui renforce le cycle de Krebs.

Les vitamines A, E, C et la vigne riche en OPC (Oligomères Procyanidoliques anti-oxydants) ont des propriétés antiradicalaires et anti-oxydantes agissant à tous les niveaux de l'oxydation. Palmaria palmata, Chondrus crispus et Fucus vesiculosus sont riches en pigments antiradicalaires et anti-oxydants.

La détoxication et la défense anti-radicalaire permettent à l'organisme de diminuer le taux des LDL oxydés et d'en permettre leur l'élimination, de neutraliser les dérivés actifs de l'oxygène.

Pour favoriser l'augmentation de la perméabilité des membranes cellulaires, on privilégie la présence:
- des éléments trace : calcium, sodium, magnésium, qui permettent une augmentation de l'assimilation et une meilleure évacuation du cholestérol par régulation des canaux ioniques; et/ou
- des huiles de colza, pépins de raisin(s), et poissons des mers froides, dont les acides gras poly-insaturés (AGPI) participent à l'augmentation de la fluidité membranaire de la cellule facilitant les échanges intra- et extra-cellulaires du cholestérol

Pour favoriser une action sur le métabolisme basal régulant une partie de la combustion et de la vitalité cellulaire, on privilégie la présence:
■ d'éléments de vitalité cellulaire de base : sodium, magnésium, calcium, fer,
■ de Palmaria palmata et Fucus vesiculosus algues riches en éléments trace : iodine participant à la régulation de la combustion cellulaire
■ et des vitamines A et PP.

Pour favoriser une action sur la transformation des acides gras poly-insaturés, on privilégie la présence:
■ d'une composante vecteur huileux, qui contient des acides gras mono-insaturés et poly-insaturés qui respectent un bon équilibre dans la composition omega3 / omega 6 : huile d'olive, huile d'onagre, pépins de raisin, colza ;
■ de zinc, fer et levures enrichies en sélénium, pour stimuler les élongases et désaturases.

Comme décrit plus haut, la composition selon l'invention active la consommation par les muscles des lipides et garantit une meilleure tonicité et un meilleur dynamisme général. Elle favorise par ailleurs l'augmentation du métabolisme basal et le contrôle du poids.

L'invention est décrite plus en détail par les exemples ci-après. D'autres aspects et avantages de la présente invention apparaîtront à la lecture des exemples, qui doivent être considérés comme illustratifs et non limitatifs.

### EXEMPLES

### A) Procédé de Fabrication :

Le procédé de fabrication est calqué sur la structure de la formule de la composition selon l'invention dont l'architecture est l'expression d'une stratégie nutritionnelle qui a pour objectif d'apporter les nutriments nécessaires ou utiles aux sous-systèmes participant directement ou indirectement au métabolisme des lipides.
Quatre principaux sous-systèmes constituent les bases sur lesquelles s'articulent la formulation et les ingrédients qui la composent. A chacun de ces sous-systèmes (ou modules) correspond une formulation qui est constituée en tant que telle, puis assemblée avec les autres modules représentant ces sous-systèmes.

### 1- Articulation de la stratégie nutritionnelle : 4 modules

- 1^{er} module :
   fonction détoxication : stimulation et régulation des cellules hépatiques, de la vésicule biliaire et de l'intestin grêle. Cette fonction est la première action de prévention et de régulation métabolique lipidique
   et défense antiradicalaire agissant sur la diminution de l'oxydation des LDL
- 2^{ème} module :
   augmentation de la perméabilité des membranes cellulaires
- 3^{ème} module :
   métabolisme basal régulant une partie de la combustion et de la vitalité cellulaire
- 4^{ème} module :
   régulation du métabolisme des lipides

### 2- Composition en rapport avec chacun des éléments et Justification de la présence des ingrédients :

- 1^{er} module : fonction détoxication et défense antiradicalaire:
   participent sur le plan des plantes, algues et champignons : ail, vigne; mycélium shiitaké; algues
   participent sur le plan vitaminique : vitamines A, B1, C, E
   L'ail et le mycélium de Shiitake présentent des propriétés de détoxication des cellules hépatiques ; ainsi que la vigne qui améliore la fonction vésiculaire et la vitamine B1 qui renforce le cycle de Krebs.
   Les vitamines A, E, C et la vigne riche en OPC possèdent des propriétés antiradicalaires et anti-oxydantes agissant à tous les niveaux de l'oxydation. Les algues marines (Palmaria palmata, Chondrus crispus et Fucus vesiculosus) sont riches en pigments antiradicalaires et anti-oxydants.
   La détoxication et la défense antiradicalaire permettent à l'organisme de diminuer le taux des LDL oxydés et d'en permettre leur l'élimination.
- 2^{ème} module : perméabilité des membranes cellulaires :
   participent sur le plan minéral : calcium, sodium, magnésium
   participent sur le plan des huiles végétal ou animales : huiles de colza, de pépins de raisin, de coprah et de poisson des mers froides
   Les éléments trace (calcium, sodium, magnésium) permettent une augmentation de l'assimilation et une meilleure évacuation du cholestérol par régulation des canaux ioniques.
   Les huiles (colza, pépins de raisin et poissons des mers froides) : les AGPI participent à l'augmentation de la fluidité membranaire de la cellule facilitant les échanges intra- et extra-cellulaires du cholestérol.
- 3^{ème} module : métabolisme basal :
   participent sur le plan minéral : calcium, sodium, magnésium, fer
   participent sur le plan des algues : algues marines (Palmaria et Fucus)
   participent sur le plan vitaminique : vitamines A, PP
   Calcium, sodium, magnésium, fer sont des éléments de vitalité cellulaire de base.
   Les algues marines (Palmaria palmata, Fucus vesiculosus) sont riches en éléments trace (tout particulièrement l'iodine) participant à la régulation de la combustion cellulaire, ainsi que les vitamines A et PP.
- 4^{ème} module : métabolisme des lipides :
   participent sur le plan minéral : zinc, fer
   participent sur le plan des huiles végétal et levures: huiles d'olive, d'onagre, de pépins de raisin, de colza ; levures enrichies
   Les composants vecteur huileux (huiles d'olive, d'onagre, de pépins de raisin, de colza) contiennent des acides gras mono-insaturés et poly-insaturés respectant un bon équilibre dans la composition omega3 / omega6.
   Zinc, fer et levures enrichies en sélénium participent à la stimulation des élongases et des désaturases.

### Support et modalités de manipulation des ingrédients :

Les ingrédients sont utilisés sous forme sèche (poudre, sels minéraux, etc) ou sous forme liquide (hydro-alcoolique ou aqueuse) pour les catégories suivantes : minéraux, extraits végétaux et apparentés (champignons, algues), vitamines.
Selon la forme galénique choisie, en fonction de leur liposolubilité ou hydrosolubilité, les substances sont traitées en émulsion huile/eau ou eau/huile.
Pour les formes sèches, les différentes solutions correspondantes à chacun des modules sont imprégnées sur le support en couches successives.
Pour la forme huileuse, un mix à 10% des 4 huiles (colza, olive, pépins de raisin, onagre) sert de base à l'intégration des nutriments composant le produit.

Chaque élément (oligo-éléments, végétaux ou apparentés, vitamines) est préparé séparément.
La solution d'ingrédients est préparée selon un procédé d'ajout successif de chaque ingrédient en veillant à l'homogénéisation de la solution à chaque étape. Entre chaque nouvel ajout la solution est soumise à dynamisation.
Chaque module défini lors de la stratégie nutritionnelle est ainsi constitué. Puis ils sont alors ajoutés les uns après les autres selon la même méthode pour aboutir à la solution finale.

### Propriétés individuelles des ingrédients

### Oleum Brassica napus oleifera - Huile de Colza

Constituée de 98% de triesters d'acides gras ; les 2 % restant sont riches en stérols et tocophérols (dont la vitamine E).
Riche en acide alpha-linolénique, acides gras poly-insaturés Oméga 3, en acides gras mono-insaturés Oméga 6 (avec un rapport intéressant Omega3:6 de 1:2,5) ; et seulement 6 à 8% d'acides gras saturés.

### Oleum Olea europea - Huile d'Olive

Riche en acide oléique : acide gras mono-insaturés (>75%), Oméga 6 (8%); l'huile d'olive contient des vitamines A, E et K. Le rapport vitamine E / AGPI est le plus élevé de toutes les huiles.

### Oleum Vitis vinifera - Huile de pépins de Raisin

Equilibrée en acide linoléique (alpha-linoléique et béta-linoléique), en acides oléique, palmitique et stéarique. Plus de 70% d'Omega 6. Fortement insaturée : le rapport poly-insaturés/saturés > 5.

### Oleum Oenothera biennis - Huile d'Onagre

Oméga 6 équilibrée en acide linoléique, en acide gamma-linolénique ; en acides oléique et stéarique.

**Oleum Pisci mare fresca - Huile de Poissons des mers froides** Riche en acide gras de la série Oméga 3. Les acides gras Oméga 3 diminuent le taux sanguin des triglycérides en diminuant la synthèse hépatique des triglycérides, en diminuant les VLDL dans le sang et leur richesse en triglycérides, ce qui permet un métabolisme plus rapide.
Les Oméga 3 permettent une bonne fluidité membranaire.

### Natrum - Sodium

Le Sodium permet la régulation acide-base de l'organisme et du métabolisme cellulaire. Le Sodium joue un rôle déterminant dans la dépolarisation cellulaire qui est à l'origine de l'excitabilité et de la conduction des influx (en particulier neuromusculaire et cardiaque), dans le maintien de l'équilibre acido-basique, de la pression osmotique, dans l'équilibre des échanges liquidiens et ioniques de l'organisme.

### Magnésium - Magnésium

Le Magnésium est indispensable à l'équilibre des canaux ioniques. Il agit comme cofacteur enzymatique et module les systèmes de transport du NA+ et du K+ dans tous les tissus ; il est le régulateur physiologique du Calcium dans l'équilibre des échanges cellulaires.
Le Magnésium joue un rôle de stabilisateur des différents organites intracellulaires : il stabilise les ribosomes qui produisent les protéines, maintient la production d'énergie par les mitochondries car indispensable à la synthèse des molécules d'A.T.P. Cette production d'énergie est la base de tous les mécanismes de vie cellulaire et de la vitalité globale de l'organisme.
Le Magnésium est indispensable à la synthèse des protéines fondamentales à la construction cellulaire (certains acides aminés, ADN et ARN).

### Calcarea - Calcium

Le Calcium est impliqué dans de nombreuses réactions enzymatiques.
Le Calcium permet la transmission des informations au niveau cellulaire en tant que second messager ce qui induit la transmission de l'influx nerveux, la contraction musculaire (par la formation d'actinomyosine), la stimulation des cellules sécrétantes (hormones comme l'insuline) et la libération des neuromédiateurs.
L'équilibre du calcium et du magnésium dans les liquides intra- et extra-cellulaires est indispensable à une bonne répartition des ions.

### Zincum - Zinc

Le Zinc intervient dans l'activité de près de 200 enzymes (en particulier dans des systèmes enzymatiques comme les oxydoréductases, l'alcool désydrogénase, la cytochrome réductase et la SOD). Les enzymes concernés par le Zinc ont une importance métabolique considérable: la glycolyse, la voie des pentoses, la néoglucogénèse, le métabolisme des lipides, et des acides gras.
Le Zinc est un métal activateur de la plupart des coenzymes nécessaires au métabolisme énergétique.
Le Zinc joue un rôle très important dans l'équilibre acido-basique (anhydrase carbonique), dans la différenciation cellulaire, dans la défense endogène anti-radicalaire. Le Zinc est un cofacteur hormonal (hormone de croissance, thyroïde, cortico-surrénales) et est indispensable à la transcription de la chaîne d'ADN (ARN-polymérase).

Le Zinc stabilise les membranes cellulaires en se couplant avec les groupements thiols en leur évitant ainsi de réagir avec le fer évitant ainsi une production de H2O2 radical libre très instable. Il intervient particulièrement dans le métabolisme de la Vitamine A (mobilisation au niveau du foie, formation du rétinol).

### Ferrum - Fer

Composant des cytochromes le fer est indispensable à la détoxication, il est indispensable à la fabrication des hormones thyroïdiennes.
Le Fer est un oligo-élément indispensable à l'état de traces, stocké dans l'organisme sous forme de ferritine et d'hémosidérine dans la moelle osseuse, le foie et la rate.
Le Fer joue un rôle prépondérant dans la constitution d'éléments indispensables à la vie. Il fait partie de sites actifs de protéines (dites " protéines à fer ") qui possèdent un rôle majeur dans l'organisme : l'hémoglobine, la myoglobine, et les cytochromes.

### Saccharomyces cerevisiae - Levures (enrichies en Sélénium)

Le Sélénium site actif de la glutathion peroxydase possède des propriétés anti-oxydantes.

### Cocos nucifera - Coprah

Riche en acide gras. Régulateur intestinal.

### Crithmum maritimum - Criste marine

Très riche en minéraux : zinc, fer, magnésium, cuivre et manganèse, en vitamines A, E, B1, B2. Possède une action détoxicante.

### Allium sativum - Ail

Riche en vitamine C, zinc, et manganèse : action hypocholestérolémiante.
L'ail se caractérise par la présence de substances soufrées originales (trisulfure d'allyle, ajoène E) ayant des effets bénéfiques sur la fluidité sanguine (réduction de l'aggrégation plaquettaire) et le taux de cholestérol sanguin (diminution de la synthèse des triglycérides) : intérêt sur le plan cardio-vasculaire.

### Palmaria palmata - Dulse

Très riche en provitamine A, capitale dans le contrôle hormonal et en vitamine C pour l'action antiradicalaire. Riche en acides aminés essentiels.

### Fucus vesiculosus - Varech vésiculeux

Riche en fucostérol : stérol qui présente des propriétés hypocholestérolémiantes comme le béta-sitostérol des végétaux.
Il contient :
- des oligo-éléments : très riche en iode et en fer ; sélénium, manganèse, cuivre, chrome, zinc
- des vitamines : C, B1, B2, B6, B 12
- des principes actifs : alginates, composés phénoliques.

### Chondrus Crispus - Carragaheen (Mousse d'Irlande)

Riche en acides gras et équilibré entre les Oméga 3 et Oméga 6 ainsi qu'en acides gras insaturés permettant l'assimilation du cholestérol.
Riche en acides aminés, oligo-métaux (particulièrement Iode, Zinc et Fer); contient toutes les vitamines.

### Lentinus edodes - Shiitake (Mycelium)

Le mycélium shiitake est très riche en acides aminés, oligo-éléments et vitamines. Il présente des propriétés hypocholestérolémiantes et pro-immunitaires.

### Vitis vinifera - Raisin (Vigne)

Le raisin est très riche en vitamines A, C et du groupe B, ainsi qu'en sels minéraux : magnésium, potassium, calcium. Le raisin draine la vésicule biliaire et le foie. Il est très riche en antiradicaux libres.

### Vitamine A (rétinol):

Le rétinol est estérifié à l'intérieur de la cellule intestinale, incorporé aux chylomicrons, excrétés dans la lymphe et rejoint la circulation générale par le canal lymphatique. La vitamine A stabilise les membranes cellulaires, la biosynthèse et la régulation des hormones stéroïdes. La synthèse de certaines protéines est sous la dépendance de la vitamine A

### Vitamine B1 (thiamine):

La vitamine B1 fournit la cellule en acide NADPH2 qui a une importance primordiale dans la synthèse des lipides, stérols et acides gras. C'est un des chaînons indispensable de la néolipidogénèse.

### Vitamine B9 (acide folique):

La vitamine B9 joue un rôle important dans le métabolisme de là sérine qui est transformée en Acétyl-coenzyme A

### Vitamine C (acide ascorbique):

La vitamine C joue un rôle biochimique important aux stades initiaux du métabolisme des lipides en association avec diverses hydroxylases. Ces hydroxylases dépendantes du cytochrome P 450 microsomal catalysent en présence de l'acide ascorbique la transformation du cholestérol en acides biliaires.

### Vitamine E (αtocophérol):

L'alpha-tocophérol accompagne les chylomicrons dans les canaux lymphatiques jusqu'à la circulation générale
Dans le plasma l'alpha-tocophérol est lié aux diverses catégories de lipoprotéines : aux LDL qui portent 40 à 60% des tocophérols et aux HDL qui en portent 35 %. Son taux est étroitement corrélé avec celui des lipides totaux et du cholestérol Elle présente un effet antioxydant: tamponnement des radicaux libres. Elle participe à la formation et à la structure des phospholipides membranaires et possède une action stabilisatrice sur les membranes cellulaires.

### Vitamine F (acide linoléique):

L'acide linoléique est un acide gras insaturé indispensable pour la synthèse et la protection des lipides.

### Vitamine PP/B3 (Niacine):

L'acide nicotinique a un effet hypocholestérolémiant (par stimulation de la protéine lipase ou par inhibition de la lipolyse médiée par l'AMP cyclique dans le tissu adipeux). C'est par ailleurs un acteur crucial dans le métabolisme énergétique cellulaire par son intervention dans tous les phénomènes d'oxydoréduction de l'organisme.

Il a cependant été démontré ci-après que la composition selon l'invention produit des effets synergiques inattendus et de loin supérieurs à ceux des ingrédients pris isolément qui ne permettent pas d'obtenir des résultats suffisants de manière à répondre au problème posé par l'invention..

### B) Préparation de la composition selon l'invention

### Exemple 1 : sous forme liquide

Une composition constituée des ingrédients suivants a été préparée sous forme liquide (boissons, spray, ...) :

Cette composition selon l'invention comprend pour 100g/100ml :
- de 7µg à 700µg d'huile de colza, de l'huile d'olive, de l'huile de pépins de raisin, de l'huile d'onagre,
- de 10µg à 1000µg de sodium, de magnésium et de calcium,
- de 10µg à 1000µg de zinc et le fer,
- de 7µg à 700µg de levures ou extraits de levure de *Saccharomyces cerevisiae,* enrichies en Sélénium,
- de 7µg à 700µg de Shiitake (mycélium),
- de 6µg à 600µg de Dulse (Palmaria palmata), de Varech vésiculeux (Fucus vesiculosus) et de Carragaeen (Chondrus crispus),
- de 7µg à 700µg de criste marine, de 6µg à 600µg d'ail et de vigne,
- de 8µg à 800µg de vitamines A, B1, B9, C, E, F et PP
- et de 7µg à 700µg d'huile de poisson des mers froides et de Coprah.

| **Micronutriments combinés** | **Par dose journalière proposée** | | **Par 100ml** | |
|---|---|---|---|---|
| Natrum | 0.05µg | 5µg | 10µg | 1000µg |
| Magnésium | 0.05µg | 5µg | 10µg | 1000µg |
| Calcarea | 0.05µg | 5µg | 10µg | 1000µg |
| Zincum | 0.05µg | 5µg | 10µg | 1000µg |
| Ferrum | 0.05µg | 5µg | 10µg | 1000µg |
| Saccharomyces cerevisiae | 0.035µg | 3.5µg | 7µg | 700µg |
| Oleum Brassica napus oleifera | 0.035µg | 3.5µg | 7µg | 700µg |
| Oleum Olea europea | 0.035µg | 3.5µg | 7µg | 700µg |
| Oleum Vitis vinifera | 035µg | 3.5µg | 7µg | 700µg |
| Oleum Oenothera biennis | 0.035µg | 3.5µg | 7µg | 700µg |
| Oleum pisci mare fresca | 0.035µg | 3.5µg | 7µg | 700µg |
| Cocos nucifera | 0.035µg | 3.5µg | 7µg | 700µg |
| Vitis vinifera | 0.03µg | 3µg | 6µg | 600µg |
| Allium sativum | 0.03µg | 3µg | 6µg | 600µg |
| Crithmum maritimum | 0.035µg | 3.5µg | 7µg | 700µg |
| Fucus vesiculosus | 0.03µg | 3µg | 6µg | 600µg |
| Palmaria palmata | 0.03µg | 3µg | 6µg | 600µg |
| Chondrus crispus | 0.03µg | 3µg | 6µg | 600µg |
| Lentinus edodes | 0.035µg | 3.5µg | 7µg | 700µg |
| Rétinol | 0.04µg | 4µg | 8µg | 800µg |
| Thiamine | 0.04µg | 4µg | 8µg | 800µg |
| Acide Folique | 0.04µg | 4µg | 8µg | 800µg |
| Acide Ascorbique | 0.04µg | 4µg | 8µg | 800µg |
| Tocophérol | 0.04µg | 4µg | 8µg | 800µg |
| Acide Linoléique | 0.04µg | 4µg | 8µg | 800µg |
| Nicotinamide | 0.04µg | 4µg | 8µg | 800µg |
| **Total** | **1**µ**g** | **100**µ**g** | **0.2mg** | **20mg** |
| Support galénique (eau) : QSP 100ml | | | | |

### Exemple 2 : sous forme huileuse

Une composition constituée des ingrédients cités dans l'exemple 1 sous forme huileuse ou matière grasse a été préparée comme suit :

| **Micronutriments combinés** | **Par dose journalière proposée** | | **Par 100ml** | |
|---|---|---|---|---|
| Natrum | 0.05µg | 5µg | 10µg | 1000µg |
| Magnésium | 0.05µg | 5µg | 10µg | 1000µg |
| Calcarea | 0.05µg | 5µg | 10µg | 1000µg |
| Zincum | 0.05µg | 5µg | 10µg | 1000µg |
| Ferrum | 0.05µg | 5µg | 10µg | 1000µg |
| Saccharomyces cerevisiae | 0.035µg | 3.5µg | 7µg | 700µg |
| Oleum Brassica napus oleifera | 0.035µg | 3.5µg | 7µg | 700µg |
| Oleum Olea europea | 0.035µg | 3.5µg | 7µg | 700µg |
| Oleum Vitis vinifera | 0.035µg | 3.5µg | 7µg | 700µg |
| Oleum Oenothera biennis | 0.035µg | 3.5µg | 7µg | 700µg |
| Oleum pisci mare fresca | 0.035µg | 3.5µg | 7µg | 700µg |
| Cocos nucifera | 0.035µg | 3.5µg | 7µg | 700µg |
| Vitis vinifera | 0.03µg | 3µg | 6µg | 600µg |
| Allium sativum | 0.03µg | 3µg | 6µg | 600µg |
| Crithmum maritimum | 0.035µg | 3.5µg | 7µg | 700µg |
| Fucus vesiculosus | 0.03µg | 3µg | 6µg | 600µg |
| Palmaria palmata | 0.03µg | 3µg | 6µg | 600µg |
| Chondrus crispus | 0.03µg | 3µg | 6µg | 600µg |
| Lentinus edodes | 0.035µg | 3.5µg | 7µg | 700µg |
| Rétinol | 0.04µg | 4µg | 8µg | 800µg |
| Thiamine | 0.04µg | 4µg | 8µg | 80µg |
| Acide Folique | 0.04µg | 4µg | 8µg | 800µg |
| Acide Ascorbique | 0.04µg | 4µg | 8µg | 800µg |
| Tocophérol | 0.04µg | 4µg | 8µg | 800µg |
| Acide Linoléique | 0.04µg | 4µg | 8µg | 800µg |
| Nicotinamide | 0.04µg | 4µg | 8µg | 800µg |
| **Total** | **1µg** | **100µg** | **0.2mg** | **20mg** |
| Support galénique huile (dont 10% de mélange d'huiles : colza, olive, onagre, pépins de raisin) : QSP 100ml | | | | |

### Exemple 3 : sous forme de gélule

Une composition constituée des ingrédients cités dans l'exemple 1 est préparée sous forme de gélule ou désigné également sous forme sèche (comprimés, gélules, ...) :

| **Micronutriments combinés** | **Par dose journalière proposée** | | **Par 100g** | |
|---|---|---|---|---|
| Natrum | 0.05µg | 5µg | 10µg | 1000µg |
| Magnésium | 0.05µg | 5µg | 10µg | 1000µg |
| Calcarea | 0.05µg | 5µg | 10µg | 1000µg |
| Zincum | 0.05µg | 5µg | 10µg | 1000µg |
| Ferrum | 0.05µg | 5µg | 10µg | 1000µg |
| Saccharomyces cerevisiae | 0.035µg | 3.5µg | 7µg | 700µg |
| Oleum Brassica napus oleifera | 0.035µg | 3.5µg | 7µg | 700µg |
| Oleum Olea europea | 0.035µg | 3.5µg | 7µg | 700µg |
| Oleum Vitis vinifera | 0.035µg | 3.5µg | 7µg | 700µg |
| Oleum Oenothera biennis | 0.035µg | 3.5µg | 7µg | 700µg |
| Oleum pisci mare fresca | 0.035µg | 3.5µg | 7µg | 700µg |
| Cocos nucifera | 0.035µg | 3.5µg | 7µg | 700µg |
| Vitis vinifera | 0.03µg | 3µg | 6µg | 600µg |
| Allium sativum | 0.03µg | 3µg | 6µg | 600µg |
| Crithmum maritimum | 0.035µg | 3.5µg | 7µg | 700µg |
| Fucus vesiculosus | 0.03µg | 3µg | 6µg | 600µg |
| Palmaria palmata | 0.03µg | 3µg | 6µg | 600µg |
| Chondrus crispus | 0.03µg | 3µg | 6µg | 600µg |
| Lentinus edodes | 0.035µg | 3.5µg | 7µg | 700µg |
| Rétinol | 0.04µg | 4µg | 8µg | 800µg |
| Thiamine | 0.04µg | 4µg | 8µg | 800µg |
| Acide Folique | 0.04µg | 4µg | 8µg | 800µg |
| Acide Ascorbique | 0.04µg | 4µg | 8µg | 800µg |
| Tocophérol | 0.04µg | 4µg | 8µg | 800µg |
| Acide Linoléique | 0.04µg | 4µg | 8µg | 800µg |
| Nicotinamide | 0.04µg | 4µg | 8µg | 800µg |
| **Total** | **1**µ**g** | **100**µ**g** | **0.2mg** | **20mg** |
| Support galénique (saccharose-lactose, saccharose-amidon, fructo-oligosaccharides, sorbitol, etc) : QSP 100g | | | | |

### C) Etude chez l'animal

### Exemule 4 : mise en évidence du rôle de la composition selon l'invention dans la baisse des taux de LDL plasmatiques.

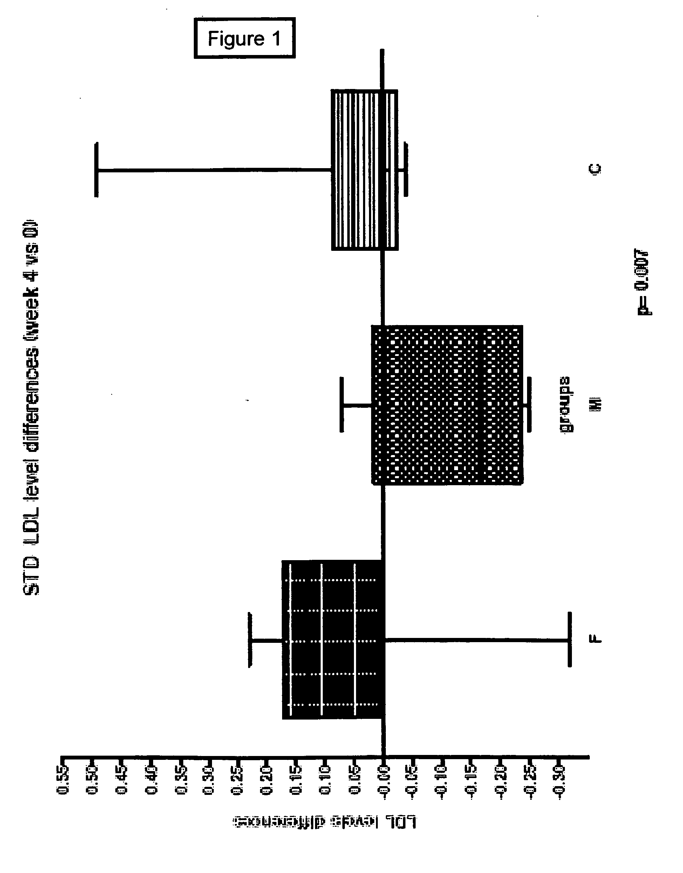

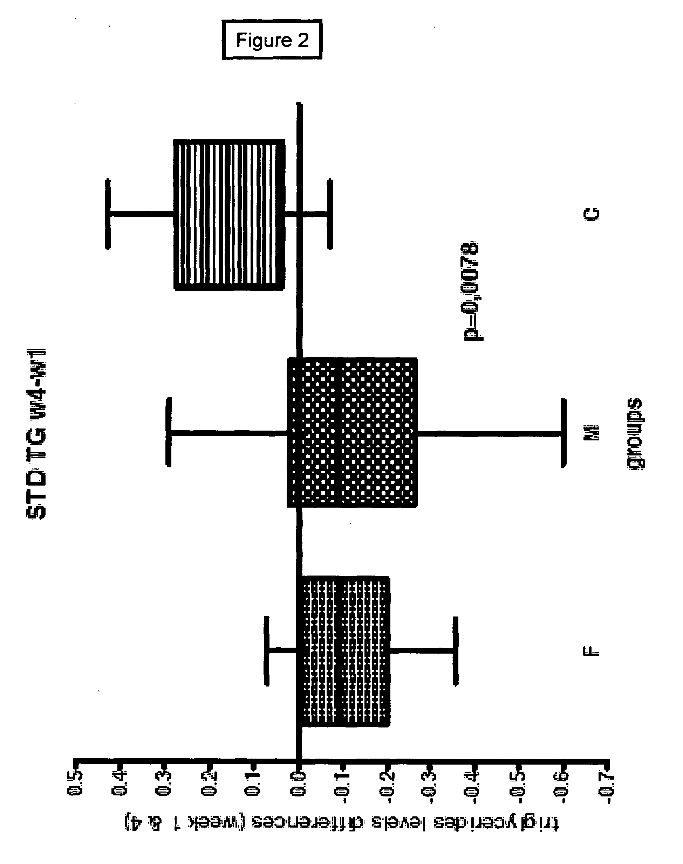

*La* *figure 1* est un graphe montrant les différences de taux de LDL plasmatiques (en mmoIL⁻¹) de souris n'exprimant pas le gène de la leptine (ci-dessous désignées souris ob/ob) soumises à un régime standard pendant 4 semaines et réparties en trois groupes : fénofibrate administré en gavage (F), composition selon l'invention incorporée à la nourriture (M), contrôle (C). La valeur p représente le degré de significativité statistique ainsi que dans la figure 2.

Chez ces souris génétiquement obèses, âgées de 8 semaines et présentant déjà à cet âge une hyperlipidémie mixte, la composition selon l'invention a fait baisser de façon significative (p=0,007) le LDL- cholestérol par rapport au contrôle, alors que le fénofibrate était inefficace dans la réduction de ce taux de LDL plasmatiques.

### Exemple 5 : mise en évidence du rôle de la composition selon l'invention dans la baisse des taux de triglycérides plasmatiques.

*La* *figure 2* est un graphe représentant les différences de taux de triglycérides plasmatiques (en mmolL⁻¹) de ces mêmes souris ob/ob soumises à un régime standard pendant 4 semaines et réparties en trois groupes comme mentionnés ci-dessus.

La baisse du taux de triglycérides (TG) plasmatiques dans le groupe recevant la la composition selon l'invention (micronutrition) est identique à celle des souris ayant ingéré le fénofibrate (actif médicamenteux ciblé sur les triglycérides). La différence entre le groupe traité par la composition selon l'invention et le groupe contrôle est statistiquement significative (p=0,0078).

### Exemple 6 : mise en évidence du rôle de la composition selon l'invention dans la prévention de la formation de plaques d'athérosclérose.

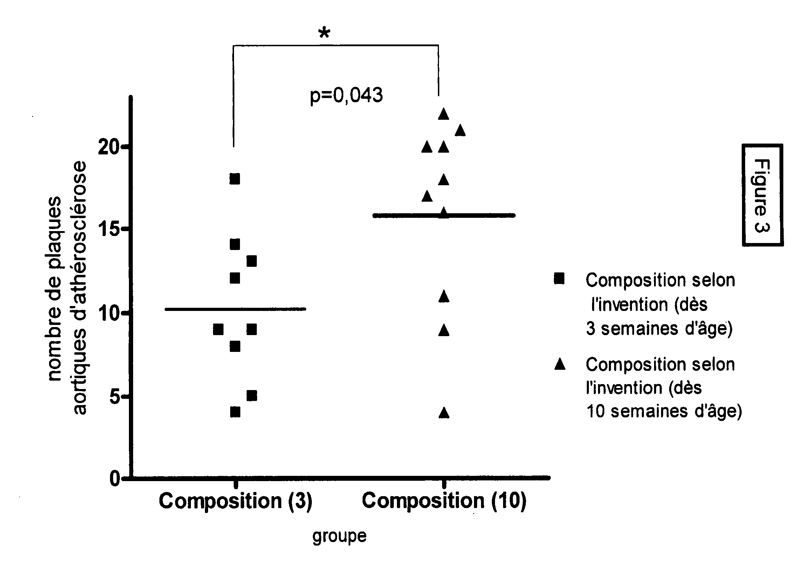

*La* *figure 3* représente le nombre de plaques aortiques d'athérosclérose à l'âge de 12 mois chez les souris mâles APOE knock-out (ko), traités dès l'âge de 3 semaines versus ceux traités depuis l'âge de 10 semaines.

Ces souris APOE ko, déficientes pour le gène de l'apo - lipoprotéine E, développent spontanément des plaques d'athérosclérose et constituent ainsi un bon modèle animal pour l'étude de l'athérosclérose humaine et surtout de l'efficacité d'un traitement dans la prévention de ce dépôt de graisses sur les parois vasculaires. Ces athéromes vasculaires obstruent les artères et sont ainsi à l'origine des maladies cardiovasculaires, dont l'infarctus du myocarde quand les artères coronaires du coeur sont oblitérées. La formation de plaques d'athérosclérose est un long processus qui progresse tout au long de la vie et commence à un âge très jeune (des stries d'athérosclérose peuvent apparaître dès l'âge de 10ans chez l'humain).
L'expérience dont le résultat est représenté par la figure 1 a pour but de vérifier si la composition selon l'invention peut diminuer la formation de plaques d'athérosclérose quand elle est administrée à un âge précoce. Deux groupes de souris mâles APOE ko, du même âge, ont reçu la composition selon l'invention incorporée dans la nourriture dès l'âge de 3 semaines (immédiatement après le sevrage) pour le premier groupe, et depuis l'âge de 10 semaines (âge adulte) pour le deuxième groupe. Les deux groupes de souris ont toujours reçu la même quantité de nourriture contenant la composition selon l'invention et ont été sacrifiées à l'âge de 12 mois. Une fois disséquées, les aortes de souris traitées dès l'âge de 3 semaines contenaient un nombre de plaques inférieur à celui retrouvé sur les aortes des souris traitées plus tardivement (depuis l'âge de 10 semaines). Cette différence étant statistiquement significative, nous pouvons donc affirmer l'efficacité de la composition selon l'invention dans la prévention de l'athérosclérose. (Le seuil de significativité statistique est admis pour une valeur p < 0,05).

### Exemple 7 : mise en évidence du rôle de la composition selon l'invention dans la prévention la stéatose hépatique.

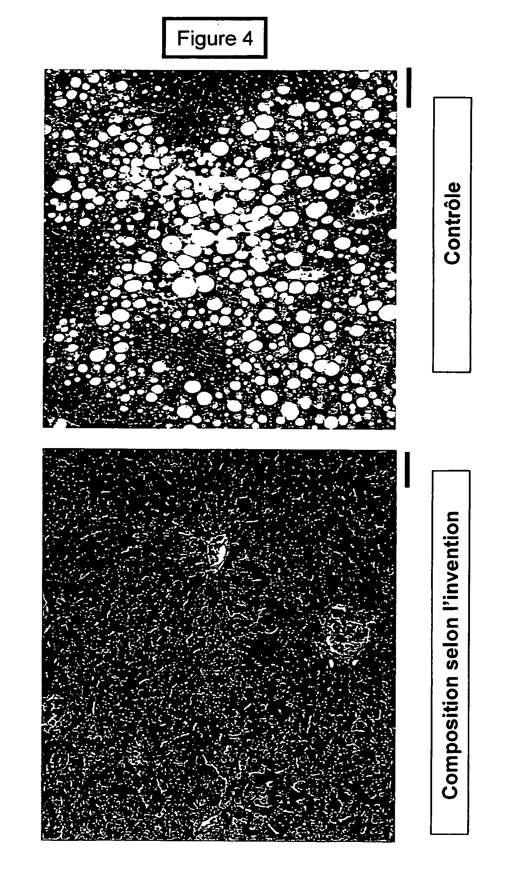

*La* *figure 4* représente l'aspect histologique du foie de souris mâles LDLr ko après 10 mois de traitement avec la composition selon l'invention versus contrôle (placebo/excipient).

Les souris LDLr ko sont déficientes pour le gène récepteur du cholestérol LDL et ont tendance à accumuler de la graisse au niveau du foie. Le foie gras ou stéatose hépatique peut évoluer vers la cirrhose qui peut se compliquer d'une insuffisance hépatique (perte des fonctions physiologiques du foie, nécessitant une transplantation du foie au stade terminal) et/ou de transformation maligne en cancer du foie. Pouvoir prévenir la stéatose hépatique est donc d'un intérêt capital sur le plan de la santé publique. L'expérience dont le résultat est représenté par la figure 4 a pour but de vérifier si l'administration continue de la composition selon l'invention peut prévenir à long terme l'accumulation de graisse dans le foie et le développement de la stéatose hépatique. Deux groupes de souris mâles LDLr ko ont reçu dès l'âge de 9 semaines, soit la composition selon l'invention incorporée dans la nourriture pour le premier groupe, soit le placebo également incorporé dans la nourriture pour le deuxième groupe. Les deux groupes de souris ont toujours reçu la même quantité de nourriture et ont été sacrifiées après 10 mois de traitement. Une fois disséqués, les foies de souris traitées ne contenaient aucun dépôt de graisse et son aspect histologique était tout à fait normal alors que les foies de souris qui ont reçu le placebo ont accumulé comme attendu les lipides sous forme de vacuoles dont est truffé le parenchyme hépatique. Devant cette différence évidente d'aspect histologique, nous pouvons donc affirmer l'efficacité de la composition selon l'invention dans la prévention de la stéatose hépatique.

### Exemple 8 : mise en évidence du rôle de la composition selon l'invention dans la prévention de l'obésité en limitant le gain de poids.

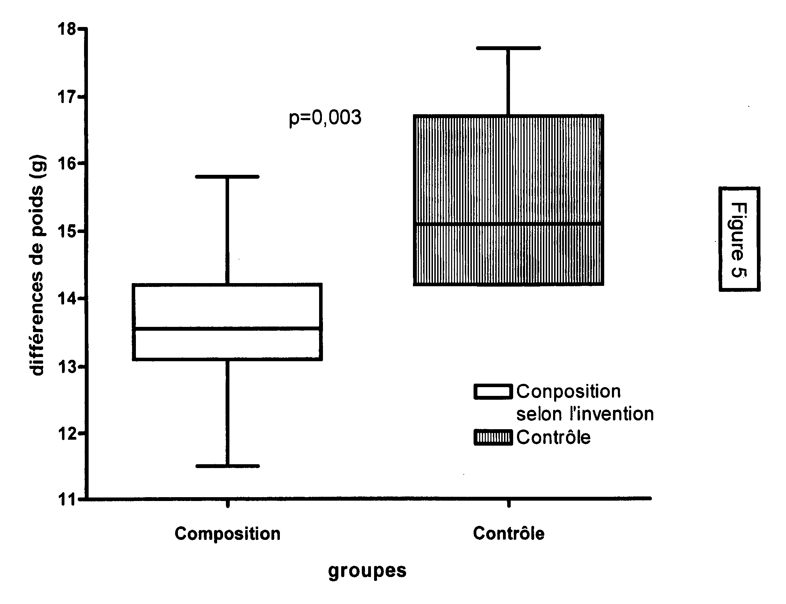

*La* *figure 5* représente le gain de poids en 4 semaines des souris OB/OB mâles recevant une nourriture enrichie à 35% en graisses (la nourriture standard utilisée pour les expériences qui précèdent et qui suivent contient 4,5% de graisses) et contenant la composition selon l'invention versus placebo.

Les souris OB/OB portent une mutation spontanée du gène de la leptine qui contrôle l'appétit et induit la satiété. Ces souris produisant une leptine mutée non fonctionnelle sont donc dans l'incapacité de se rassasier et se nourrissent en permanence. Ce trouble du comportement alimentaire est à l'origine d'une obésité sans cesse croissante et fait de ces souris un bon modèle d'étude de l'efficacité des traitements visant à contrôler le poids corporel.
Devant l'ampleur de la progression de l'obésité dans le monde et ses complications majeures pour la santé publique sur le plan cardio-vasculaire, respiratoire, hépato-biliaire, ostéo-articulaire, reproductif et psychosocial, un traitement capable de contrôler la prise de poids même avec une alimentation riche en graisses est d'un intérêt médical considérable.
L'expérience dont le résultat est représenté par la figure 3 a pour but de vérifier si l'incorporation de la composition selon l'invention dans une nourriture riche en graisses peut limiter le gain de poids induit par ce régime alimentaire.
Deux groupes de souris mâles OB/OB ont reçu dès l'âge de 9 semaines, soit la composition selon l'invention incorporée dans la nourriture pour le premier groupe, soit le placebo également incorporé dans la nourriture pour le deuxième groupe. Les deux groupes de souris ont toujours reçu la même quantité de nourriture et ont été pesées avant et après 1 mois de traitement. La différence de poids entre la semaine 4 et la semaine 0 a révélé un gain de poids qui est inférieur chez les souris traitées versus contrôle. Cette différence étant statistiquement significative, nous pouvons donc affirmer l'efficacité de la composition selon l'invention dans la prévention de l'obésité en limitant le gain de poids et en contrôlant le poids corporel.

### Exemple 9 : mise en évidence du rôle de la composition selon l'invention dans la prévention de l'obésité en stabilisant la masse graisseuse corporelle.

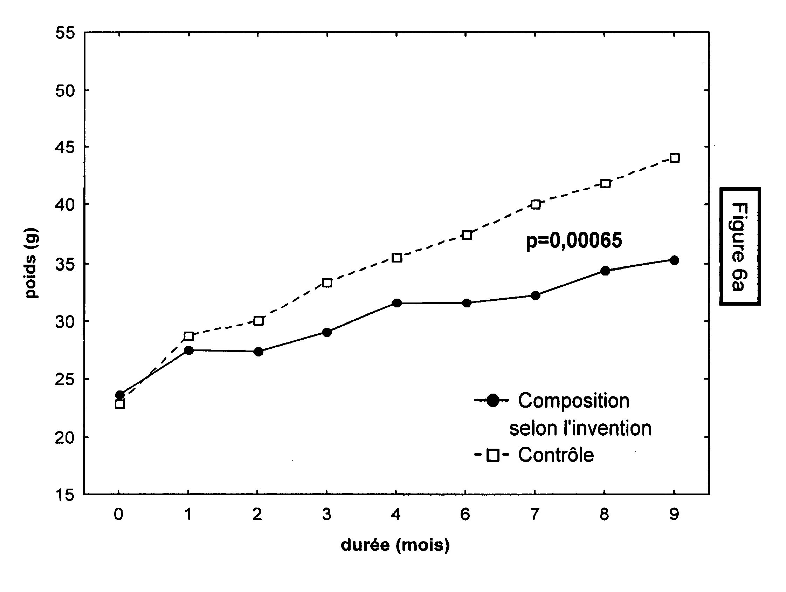

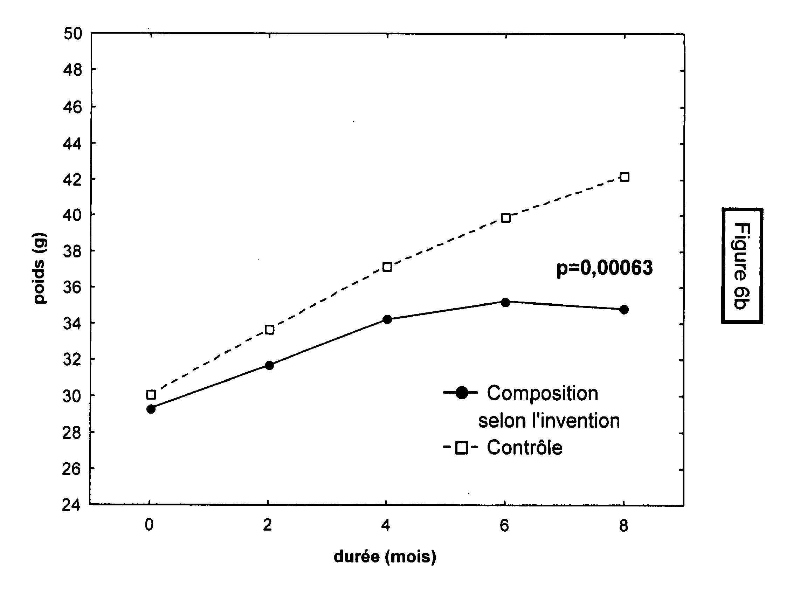

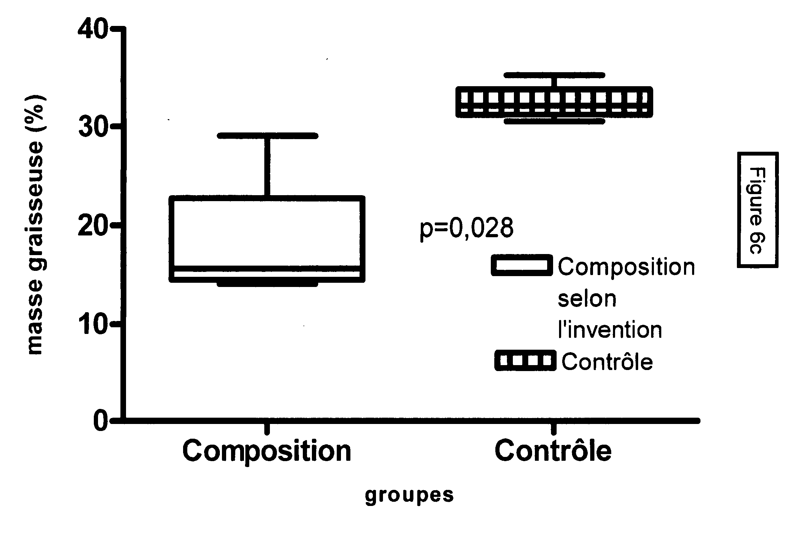

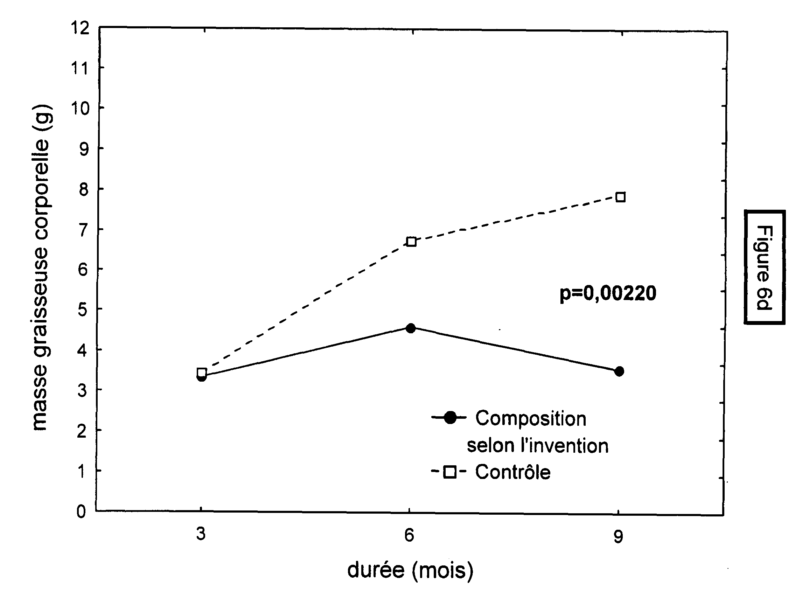

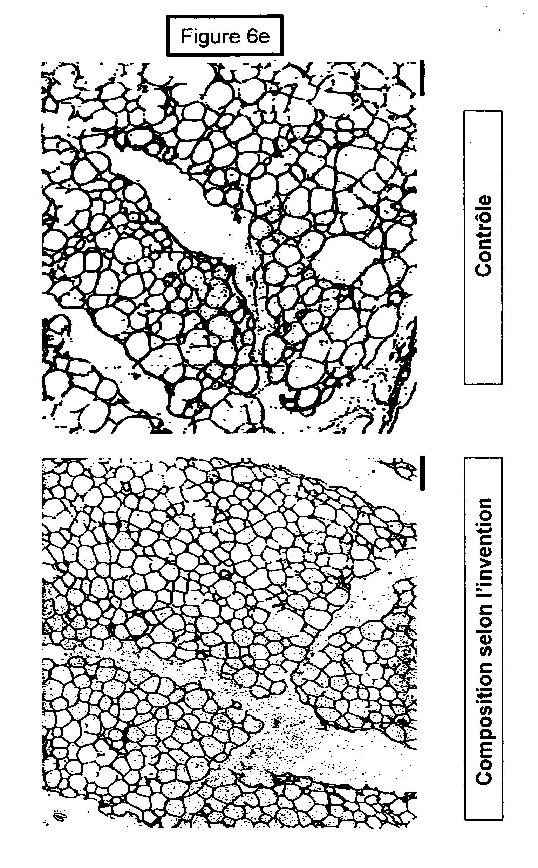

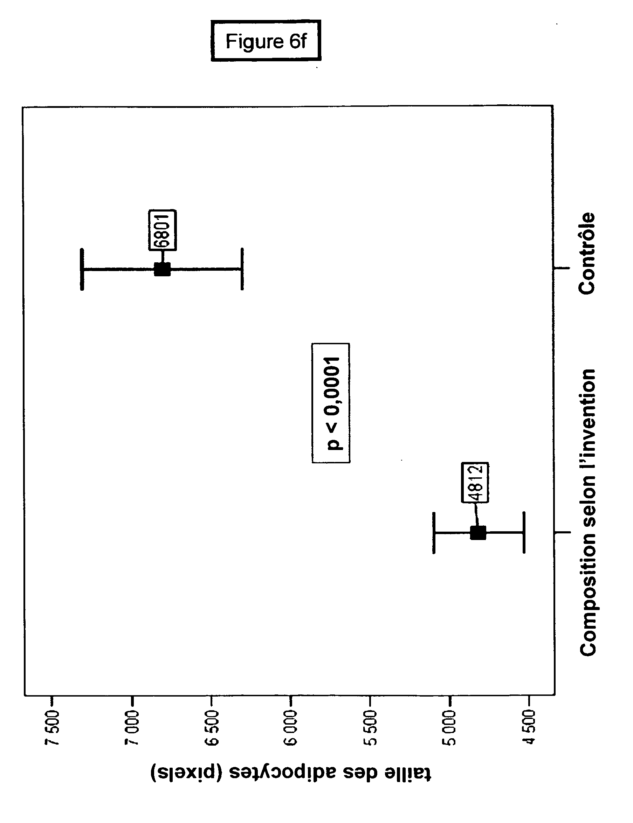

*La* *figure 6* est multiple et illustre la limitation du gain de poids chez les souris LDLr ko et APOE ko ainsi que la stabilisation de la masse graisseuse corporelle avec une limitation de la taille des adipocytes (cellules graisseuses) induites par la composition selon l'invention versus contrôle.

La limitation du gain de poids expliquée dans l'exemple 8 a été confirmée à long terme pour deux autres lignées de souris sus citées que sont les LDLr ko (figure 6a) et les APOE ko (figure 6b) et nous permet d'affirmer l'efficacité de la composition selon l'invention dans la prévention de l'obésité. Néanmoins, il était important de vérifier que la limitation du gain de poids passe effectivement par la stabilisation du tissu graisseux. Ainsi, les expériences dont le résultat est représenté par les figures 6c, 6d, 6e et 6f a pour but de documenter l'effet de la composition selon l'invention sur la masse graisseuse corporelle.
Deux groupes de souris mâles LDLrko ont reçu dès l'âge de 2 mois, soit la composition selon l'invention incorporée dans la nourriture pour le premier groupe, soit le placebo également incorporé dans la nourriture pour le deuxième groupe. Idem pour l'expérience avec les souris mâles APOE ko. Les deux groupes expérimentaux pour chaque lignée de souris ont toujours reçu la même quantité de nourriture et ont été scannés à l'EchoMRI après 10 mois de traitement pour les souris LDLr ko et après 3, 6 et 9 mois de traitement pour les souris APOE ko. La figure 6c montre chez les souris LDLr ko que la masse graisseuse corporelle du groupe traité est inférieure à celle du groupe contrôle à la fin du traitement. Plus encore, la figure 6d montre un effet certain de la composition selon l'invention sur l'évolution de la masse graisseuse au long terme. En effet, il est facile de constater sur la figure 6d que la masse graisseuse corporelle reste stable chez les souris APOE ko traitées, tandis qu'elle augmente chez les souris non traitées (contrôle). Ces différences étant statistiquement significatives, nous pouvons donc affirmer l'efficacité de la composition selon l'invention dans la prévention de l'obésité en stabilisant la masse graisseuse corporelle. Les figures 6e et 6f viennent étayer d'avantage cet effet en montrant sur le plan histologique des cellules adipeuses (constituant la masse graisseuse) plus petites et donc contenant moins de graisses, chez les souris LDLr ko traitées versus contrôle (figure 6e). La figure 6f montre que la taille moyenne des cellules adipeuses chez les souris LDLr ko traitées est bien inférieure à celle des cellules du groupe non traité (contrôle). La différence est là aussi statistiquement significative et nous permet d'affirmer que la composition selon l'invention stabilise la masse graisseuse en limitant l'accumulation des graisses dans les cellules adipeuses.

### Exemple 10 : mise en évidence du rôle de la composition selon l'invention dans l'augmentation de la consommation d'oxygène.

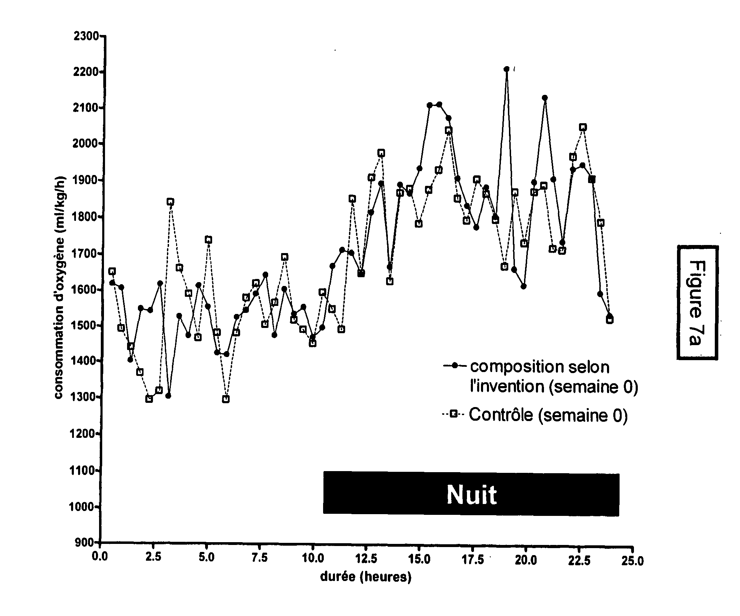

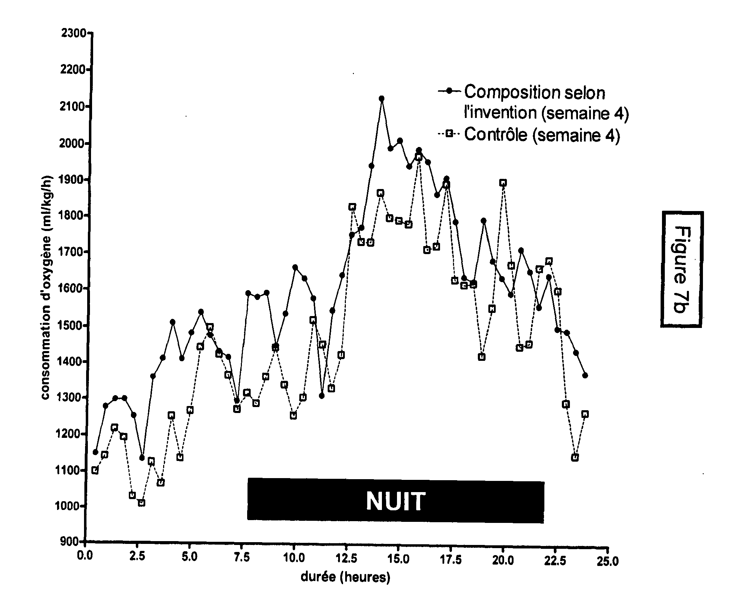

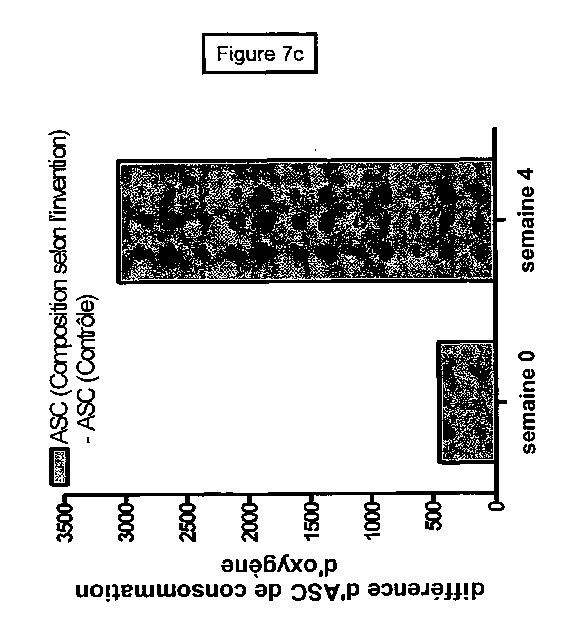

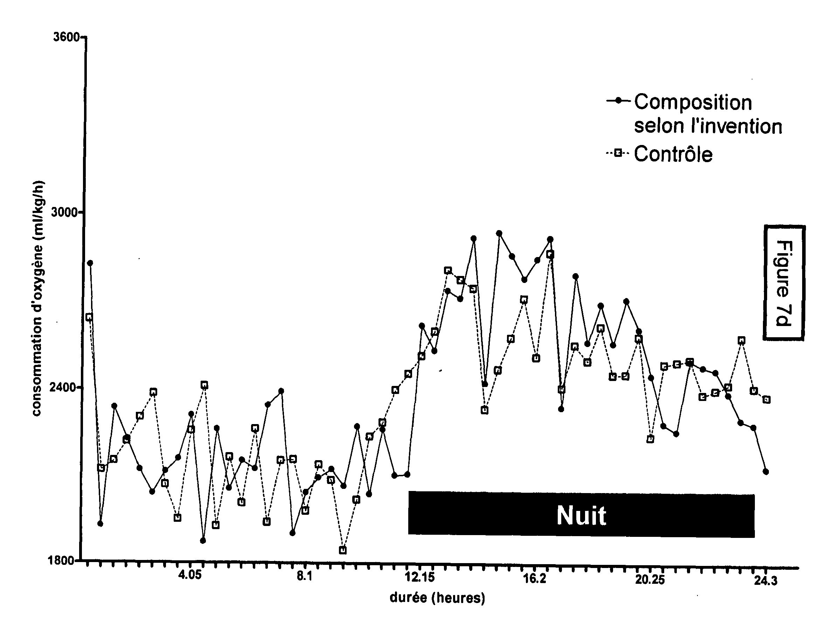

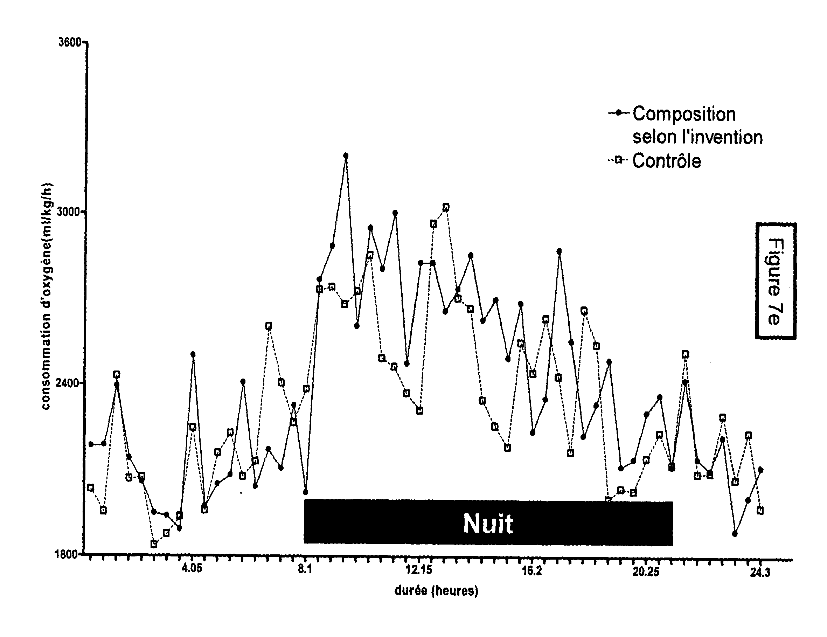

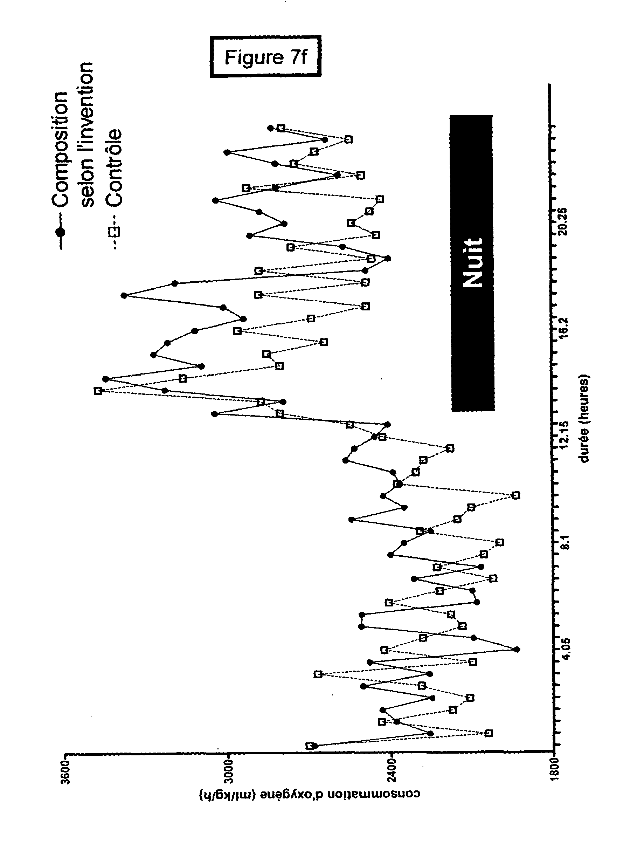

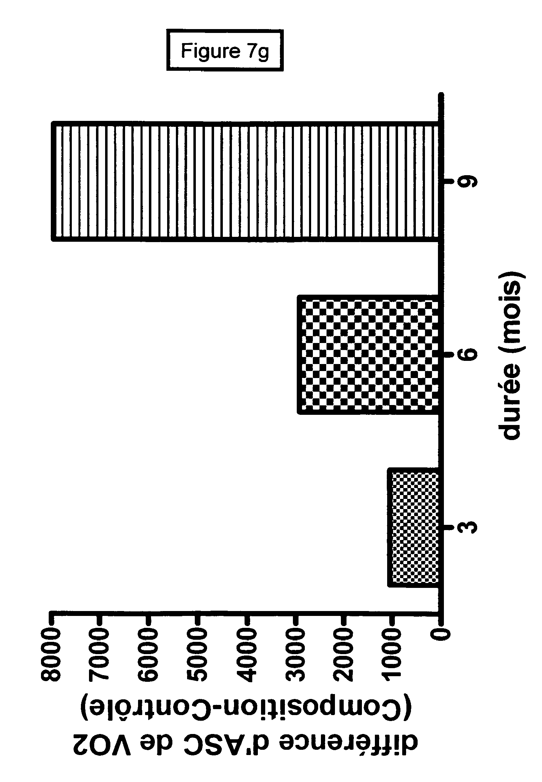

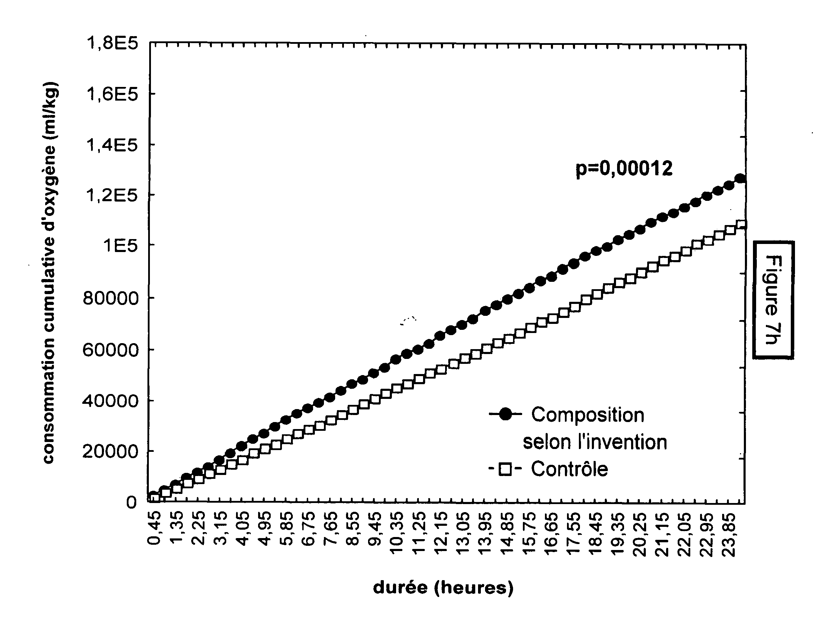

*La* *figure 7* est multiple et illustre l'augmentation de la consommation d'oxygène chez les souris OB/OB (figure 7a, b et c), APOE ko (figure 7d, e, f et g) et LDLr ko (figure 7h) induite par la composition selon l'invention versus contrôle.

L'utilisation des lipides par l'organisme comme substrat énergétique implique leur oxydation et donc une consommation d'oxygène. En accord avec les constatations de l'exemple 6 qui montre une limitation du stockage des graisses par la composition selon l'invention, la figure 7 montre que cette composition augmente la consommation d'oxygène servant à l'oxydation des lipides qui au lieu d'être stockés sont ainsi éliminés.
Deux groupes de souris mâles OB/OB ont reçu dès l'âge de 2mois, soit la composition selon l'invention incorporée dans la nourriture pour le premier groupe, soit le placebo également incorporé dans la nourriture pour le deuxième groupe. Idem pour l'expérience avec les souris mâles LDLrko et APOE ko. Les deux groupes expérimentaux pour chaque lignée de souris ont toujours reçu la même quantité de nourriture et ont séjourné dans une cage calorimétrique avant et après 1 mois de traitement pour les souris OB/OB, après 5 mois de traitement pour les souris LDLr ko et après 3, 6 et 9 mois de traitement pour les souris APOE ko. La figure 7a montre les courbes de consommation d'oxygène enregistrées pendant les 24 heures de séjour des souris dans les cages calorimétriques individuelles, spécialement équipées pour mesurer ce paramètre. Les courbes représentées sur cette figure ont été enregistrées avant le début du traitement par la composition selon l'invention. Comme le montre la figure 5a, les courbes de consommation d'oxygène des deux groupes expérimentaux avant traitement sont superposables. Après 1 mois de traitement, la courbe de consommation d'oxygène du groupe traité avec la composition selon l'invention se détache vers le haut par rapport à la courbe du groupe contrôle, comme le montre la figure 7b, et révèle ainsi une augmentation de la consommation d'oxygène induite par la composition selon l'invention. Cette constatation est très bien illustrée par la figure 7c qui montre la différence de l'aire sous la courbe de consommation d'oxygène du groupe traité et celle du groupe contrôle à la semaine 0 (avant traitement) et 4 (après 1 mois de traitement). La différence de l'aire sous la courbe entre le groupe traité et le groupe contrôle a ainsi été multipliée par 6 en faveur d'un détachement vers le haut de la courbe du groupe traité par rapport à celle du groupe contrôle.
Cette augmentation de la consommation d'oxygène induite par la composition selon l'invention a été confirmée pour les deux autres lignées de souris APOE ko et LDLr ko. De la même façon que les figures 7a et b, les figures 7d, e et f représentent les courbes de consommation d'oxygène des groupes de souris APOE ko contrôle et traité avec la composition selon l'invention à 3 mois (figure 5d), 6 mois (figure 5e) et 9 mois (figure 7f) de traitement. Sur ces figures, nous pouvons constater un détachement vers le haut progressif et constant de la courbe de consommation d'oxygène du groupe traité par rapport à celle du groupe contrôle, tout au long de ces mois de traitement. Comme la figure 7c, la figure 7g montre bien que ce détachement creuse de façon régulière l'écart entre l'aire sous la courbe de consommation d'oxygène du groupe traité et celle du groupe contrôle qui est ainsi multiplié par 3, entre le 3^{ème} et le 6^{ème} mois, puis par 8 au 9^{ème} mois. Enfin, la figure 7h montre que pour les souris LDLr ko, la composition selon l'invention augmente également leur consommation en oxygène. En effet, cette figure représente les courbes de consommation cumulative en oxygène durant 24 heures des souris du groupe traité et contrôle. La courbe du groupe traité indique que la consommation en oxygène de ce groupe est supérieure à celle du groupe contrôle. Cette différence étant statistiquement significative, nous pouvons donc affirmer que la composition selon l'invention augmente la consommation de l'organisme en oxygène.

### Exemple 11: mise en évidence du rôle de la composition selon l'invention dans l'augmentation du métabolisme oxydatif.

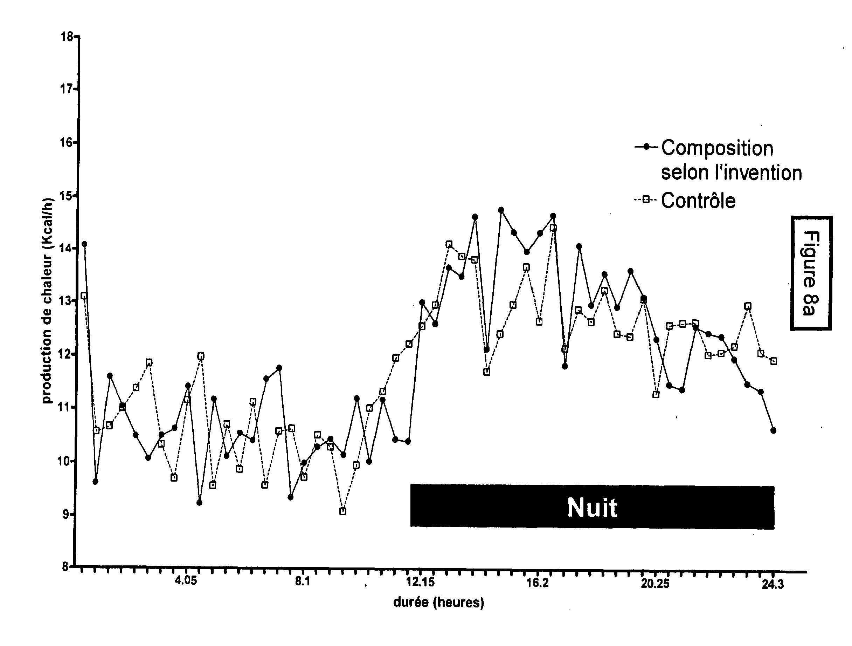

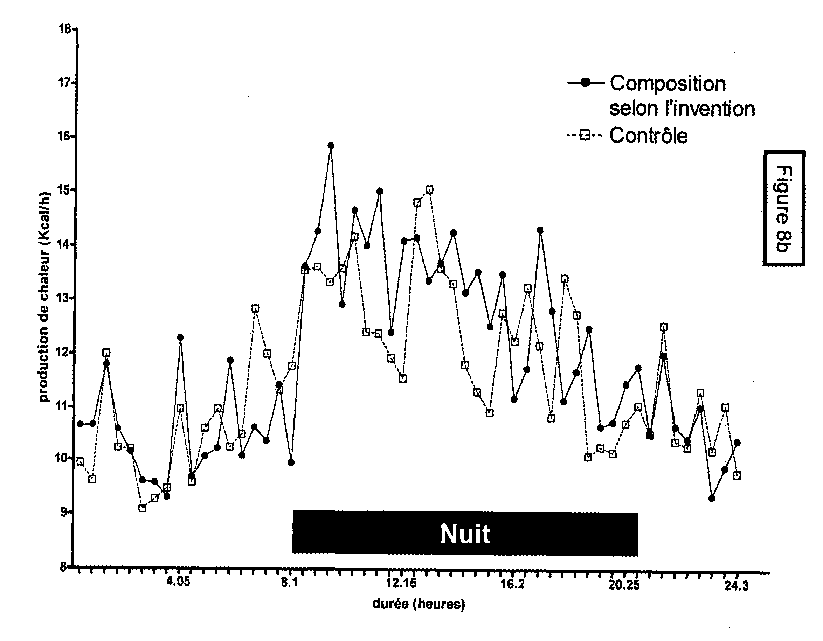

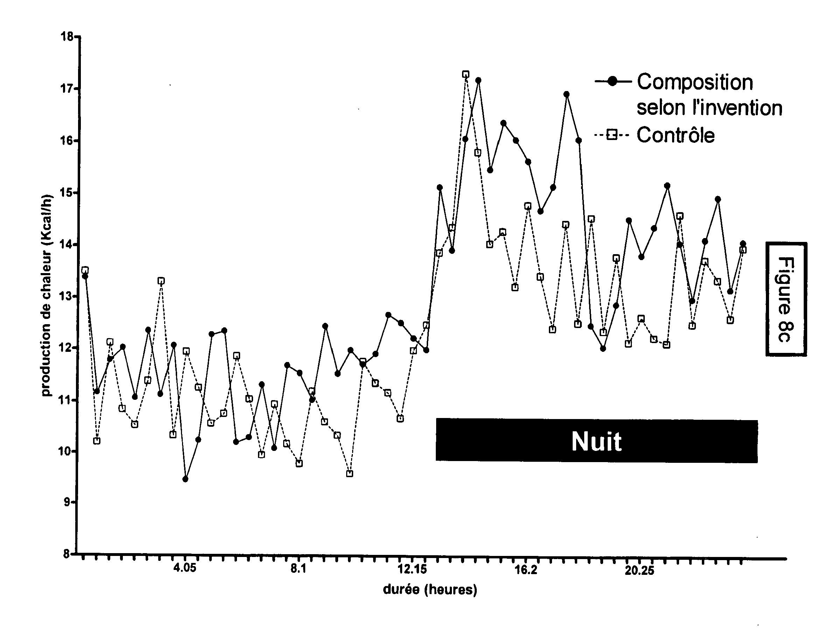

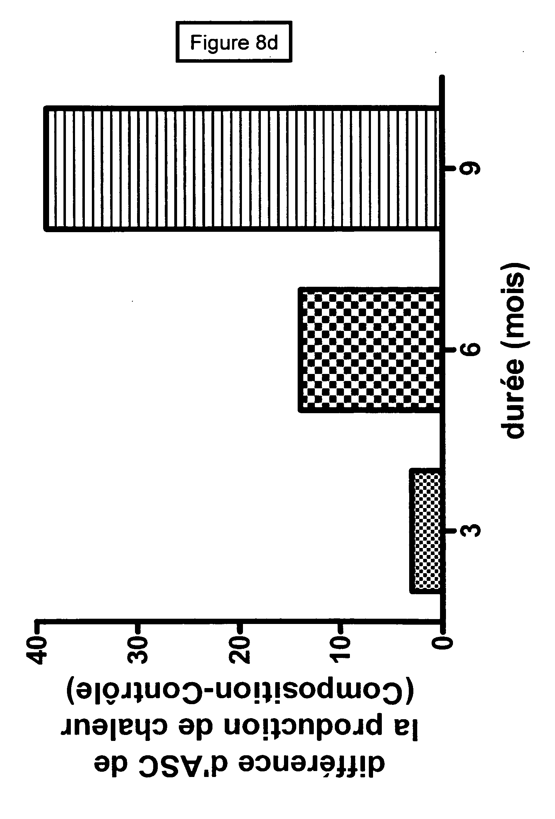

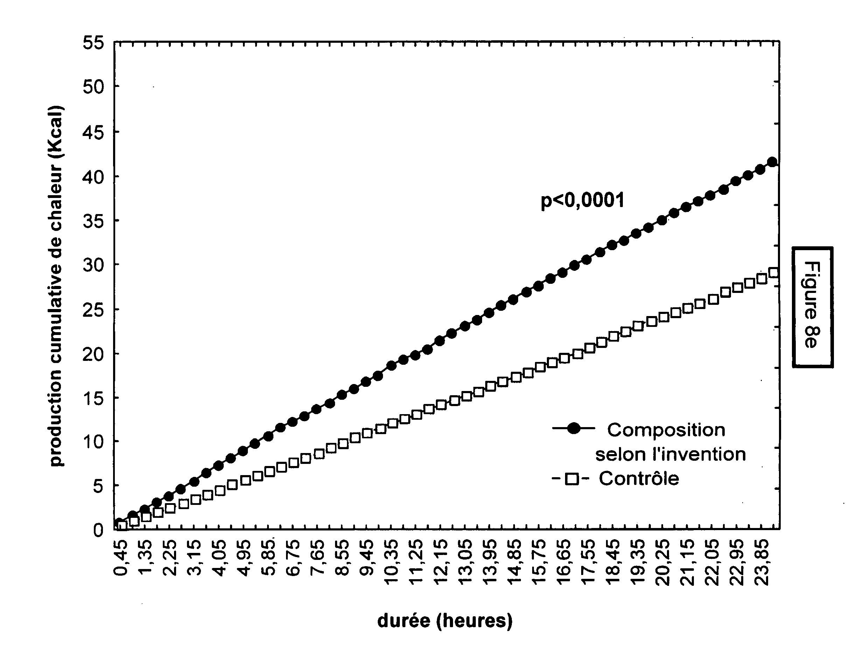

*La* *figure 8* est multiple et illustre l'augmentation de la production de chaleur par les souris APOE ko (figure 8a, b, c et d) et LDLr ko (figure 8e) induite par la composition selon l'invention versus contrôle.

L'augmentation du métabolisme oxydatif est reflétée par l'augmentation conjointe de la consommation d'oxygène et de la production de chaleur. Ainsi, en augmentant ces deux paramètres, la composition selon l'invention augmente de toute évidence le métabolisme oxydatif de l'organisme.
En effet, les valeurs de kilo calories produites ayant été obtenues en même temps et chez les mêmes animaux que les mesures du volume d'oxygène consommé, l'interprétation des figures 8 a, b, c et d peut être calquée sur celle des figures 8 d, e, f et g qui montrent un détachement vers le haut progressif et constant de la courbe de production de chaleur du groupe traité par rapport à celle du groupe contrôle, tout au long des 9 mois de traitement. Comme la figure 7g, la figure 8d montre bien que ce détachement creuse de façon régulière l'écart entre l'aire sous la courbe de production de chaleur du groupe traité et celle du groupe contrôle qui est ainsi multiplié par plus de 4, entre le 3^{ème} et le 6^{ème} mois, puis par 13 au 9^{ème} mois.
De même, la figure 8e montre que pour les souris LDLr ko, la composition selon l'invention augmente également leur production de chaleur. En effet, cette figure représente les courbes de production cumulative de chaleur durant 24 heures des souris du groupe traité et contrôle. La courbe du groupe traité indique que la production de chaleur de ce groupe est supérieure à celle du groupe contrôle. Cette différence étant statistiquement significative, nous pouvons donc affirmer que la composition selon l'invention augmente le métabolisme oxydatif en augmentant conjointement la production de chaleur et la consommation de l'organisme en oxygène (exemple 7).

### Exemple 12: mise en évidence du rôle de la composition selon l'invention dans l'augmentation de la biogenèse des crêtes mitochondriales qui sont le siège du métabolisme oxydatif.

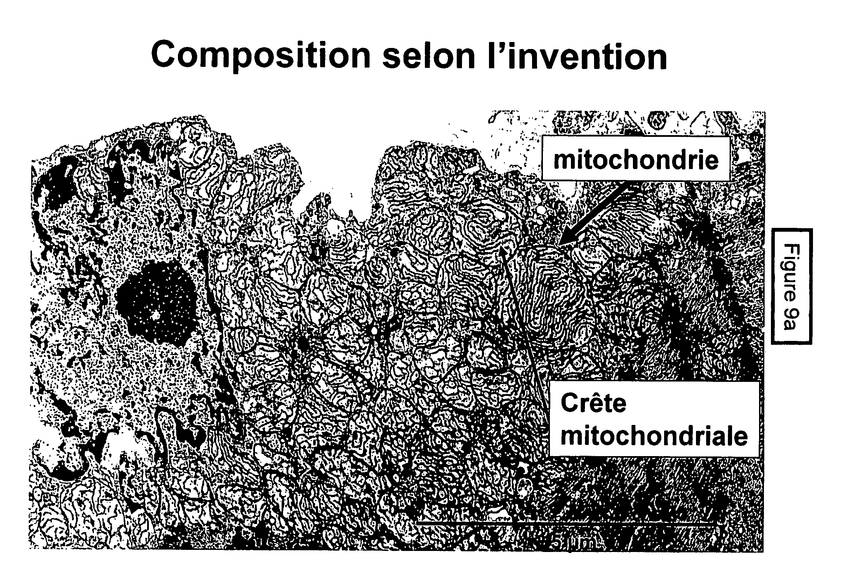

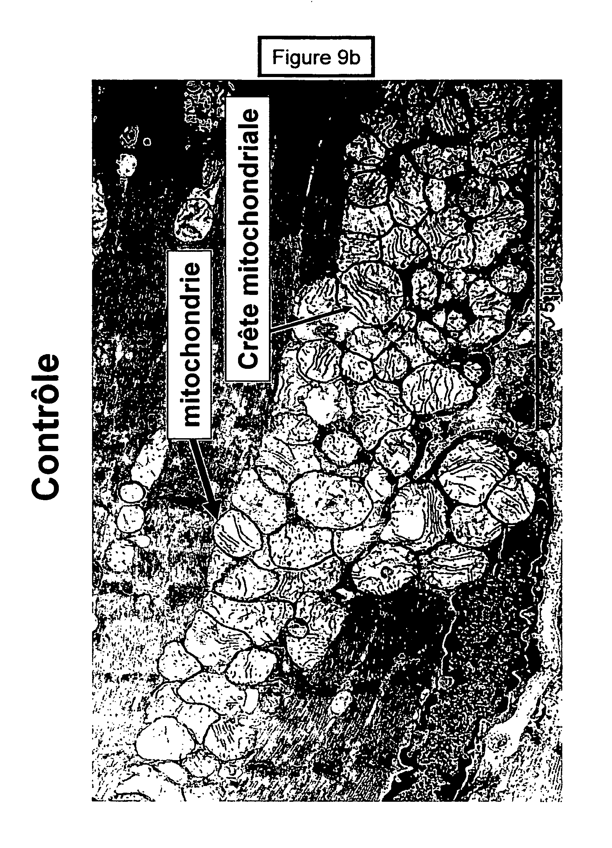

*La* *figure 9* montre une augmentation de la densité des crêtes mitochondriales chez les souris OB/OB induite après 1 mois de traitement par la composition selon l'invention versus contrôle.

Deux groupes de souris mâles OB/OB ont reçu dès l'âge de 2mois, soit la composition selon l'invention incorporée dans la nourriture pour le premier groupe, soit le placebo également incorporé dans la nourriture pour le deuxième groupe. Les deux groupes expérimentaux ont toujours reçu la même quantité de nourriture et ont été disséqués après 1mois de traitement afin de prélever au niveau des pattes postérieures des souris le soleus, qui est un muscle oxydatif, et d'en analyser l'ultra structure au microscope électronique.
Les mitochondries sont les unités de production d'énergie dont est équipée la majorité des cellules de l'organisme et l'oxydation des lipides a lieu au niveau des crêtes mitochondriales. L'augmentation de la densité de ces crêtes chez le groupe traité (figure 9a) versus contrôle (figure 9b) nous permet d'affirmer que la composition selon l'invention augmente l'oxydation des lipides et donc leur consommation par l'organisme.

### Exemple 13: mise en évidence du rôle de la composition selon l'invention dans l'augmentation de la performance physique et de l'endurance.

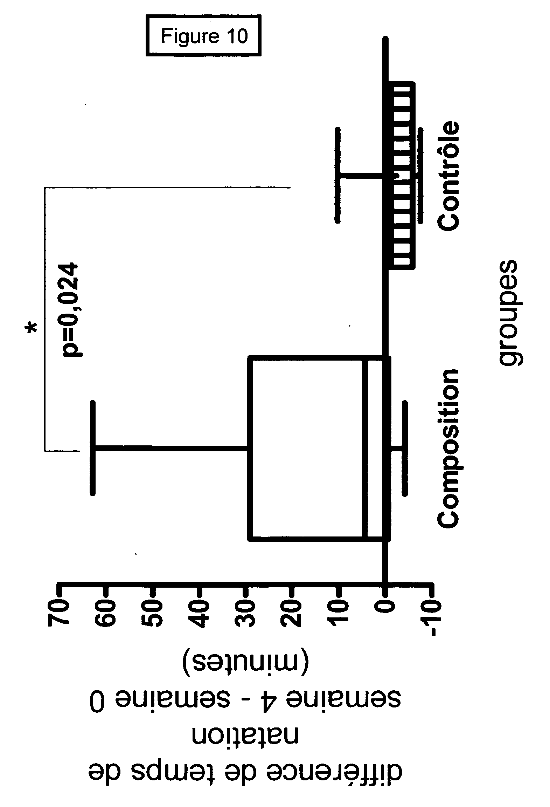

*La* *figure 10* montre une augmentation du temps de natation chez les souris OB/OB après 1 mois de traitement par la composition selon l'invention versus contrôle.

Deux groupes de souris mâles OB/OB ont reçu dès l'âge de 2mois, soit la composition selon l'invention incorporée dans la nourriture pour le premier groupe, soit le placebo également incorporé dans la nourriture pour le deuxième groupe. Les deux groupes expérimentaux ont toujours reçu la même quantité de nourriture. Les souris ont été forcées à nager grâce à un poids (7,5% du poids corporel) attaché à leurs pattes postérieures et le temps de natation a été enregistré dans ces conditions pour chaque souris avant et après 1 mois de traitement.
L'augmentation du temps de natation est reconnue comme un indice de l'augmentation de l'endurance. Ainsi, comme le montre la figure 10, nous avons pu enregistrer une augmentation du temps de natation et donc d'endurance chez le groupe de souris traitées contrairement au groupe contrôle dont le temps de natation a même diminué suivant l'évolution naturelle des performances physiques qui diminuent avec l'âge. Cette augmentation étant significative nous permet d'affirmer que la composition selon l'invention augmente l'endurance et la performance physique. L'intérêt nutritionnel de cette composition pour l'optimisation des performances sportives ainsi que pour l'amélioration et le maintien de la forme physique est donc très concret.

### Exemple 14 : mise en évidence du rôle de la composition selon l'invention dans la stabilisation du taux plasmatique des triglycérides.

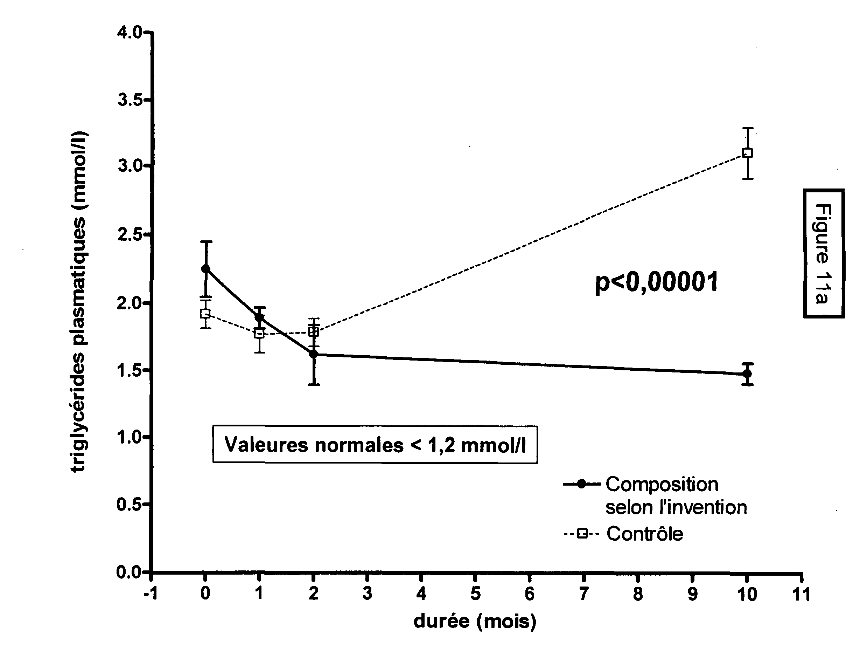

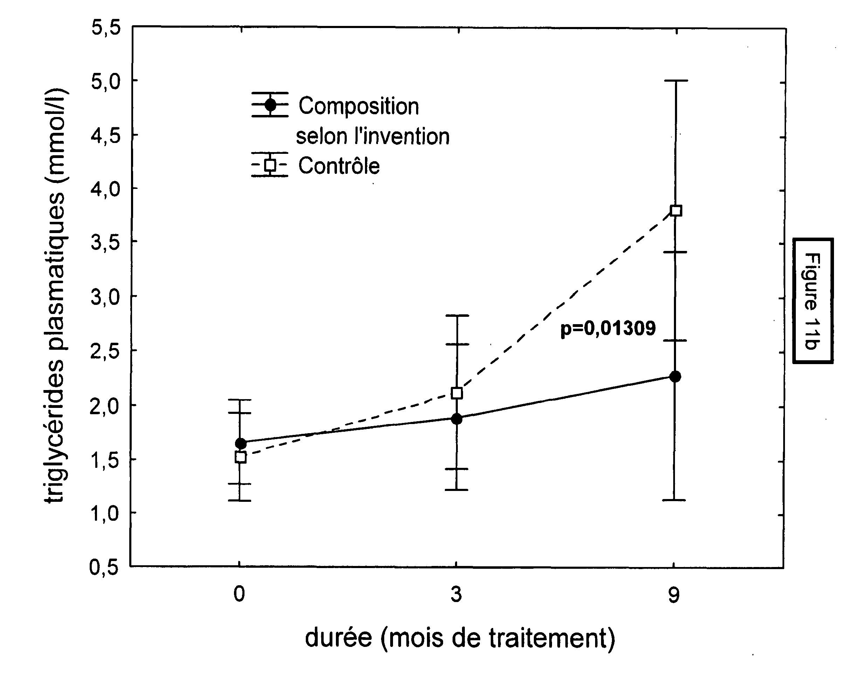

*La* *figure 11* illustre la stabilisation du taux plasmatique des triglycérides chez les souris LDLr ko et APOE ko induite par la composition selon l'invention versus contrôle.

La stabilisation sur le long terme du taux plasmatique des triglycérides est un atout majeur dans la lutte contre le syndrome métabolique et les maladies cardio-vasculaires. Ainsi, les expériences dont le résultat est représenté par les figures 11a et 11b avaient pour but de vérifier si l'effet hypolipémiant de la composition selon l'invention se maintient à long terme.
Deux groupes de souris mâles LDLrko ont reçu dès l'âge de 2mois, soit la composition selon l'invention incorporée dans la nourriture pour le premier groupe, soit le placebo également incorporé dans la nourriture pour le deuxième groupe. Idem pour l'expérience avec les souris mâles APOE ko. Les deux groupes expérimentaux pour chaque lignée de souris ont toujours reçu la même quantité de nourriture et leur sang a été prélevé par ponction rétro-orbitale avant le début du traitement puis après 1 mois, puis 2 mois puis 10 mois de traitement pour les souris LDLr ko (figure 11a) et après 3 et 9 mois de traitement pour les souris APOE ko (figure 11b). Les deux figures 11a et 11b montrent une même efficacité de la composition selon l'invention à maintenir stable les taux de triglycérides plasmatiques, sur le long terme, aussi bien chez les souris LDLr ko que APOE ko alors que les taux de souris contrôles augmentent irrémédiablement. Cette différence étant statistiquement significative nous permet d'affirmer l'efficacité hypolipémiante sur le long terme de la composition selon l'invention et donc son rôle dans la prévention du syndrome métabolique et des maladies cardio-vasculaires.

### Exemple 15 : mise en évidence du rôle de la composition selon l'invention dans le maintien du taux plasmatique des triglycérides stable en post-prandial (après le repas).

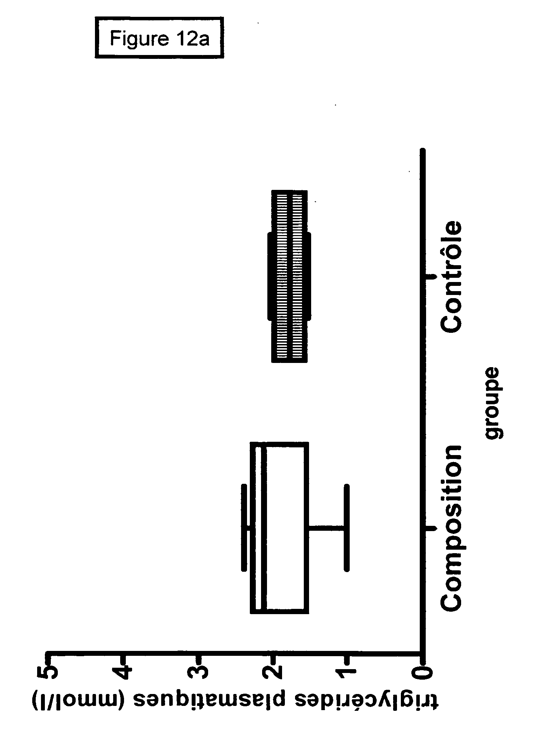

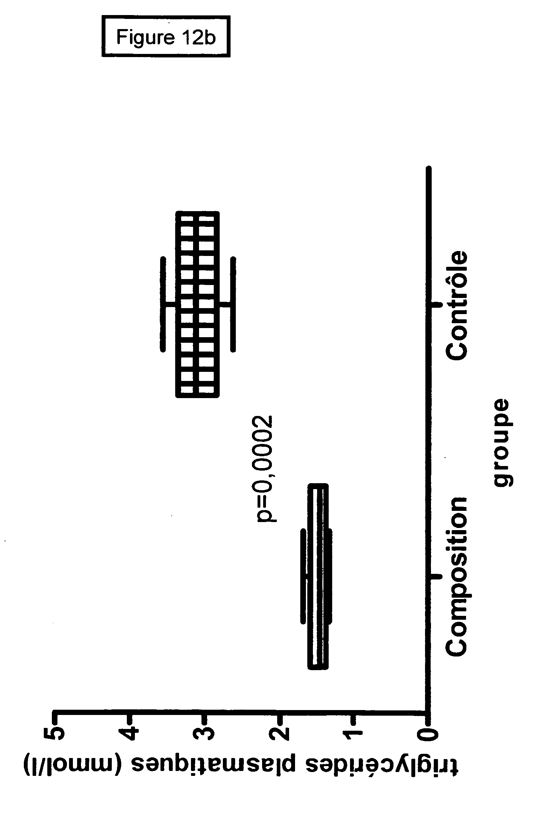

*La* *figure 12* illustre l'absence d'élévation en post-prandial du taux plasmatique des triglycérides chez les souris LDLr ko grâce à la composition selon l'invention versus contrôle.

Un des mécanismes hypolipémiants est la diminution de l'absorption intestinale de triglycérides alimentaires. De plus, la surcharge post-prandiale du plasma en triglycérides alimentaires est un facteur de risque vasculaire majeur et bien reconnu. Ainsi, en empêchant l'élévation du taux des triglycérides plasmatiques en post-prandial, la composition selon l'invention évite non seulement l'inondation de l'organisme par une surcharge lipidique mais protège également les parois vasculaires et prévient ainsi la formation de plaques d'athérosclérose (voir exemple 7).
Ainsi, l'expérience dont le résultat est représentée par les figures 12a et 12b a pour but de vérifier si l'effet hypolipémiant de la composition selon l'invention passe par une limitation de l'absorption intestinale des lipides alimentaires.

Deux groupes de souris mâles LDLrko ont reçu dès l'âge de 2mois, soit la composition selon l'invention incorporée dans la nourriture pour le premier groupe, soit le placebo également incorporé dans la nourriture pour le deuxième groupe. Les deux groupes expérimentaux pour chaque lignée de souris ont toujours reçu la même quantité de nourriture et leur sang a été prélevé après 10 mois de traitement, par ponction rétro-orbitale au terme de 16 heures de jeûne (figure 12a) puis à l'état nourri (figure 12b). La figure 12a montre des taux de triglycérides plasmatiques équivalents à jeun pour les deux groupes expérimentaux. La figure 12b montre par contre des taux différents de triglycérides plasmatiques à l'état nourri pour les deux groupes expérimentaux, avec une élévation importante pour le groupe contrôle qui a été évitée chez le groupe traité par la composition selon l'invention. Cette différence étant statistiquement très significative nous permet d'affirmer l'efficacité de la composition selon l'invention à maintenir une triglycéridémie stable en post prandial et donc son rôle dans la prévention du syndrome métabolique et des maladies cardio-vasculaires.

### Exemple 16 : mise en évidence du rôle de la composition selon l'invention dans l'augmentation de l'utilisation des lipides par les muscles via une activation du récepteur PPAR alpha, acteur majeur du catabolisme oxydatif lipidique.

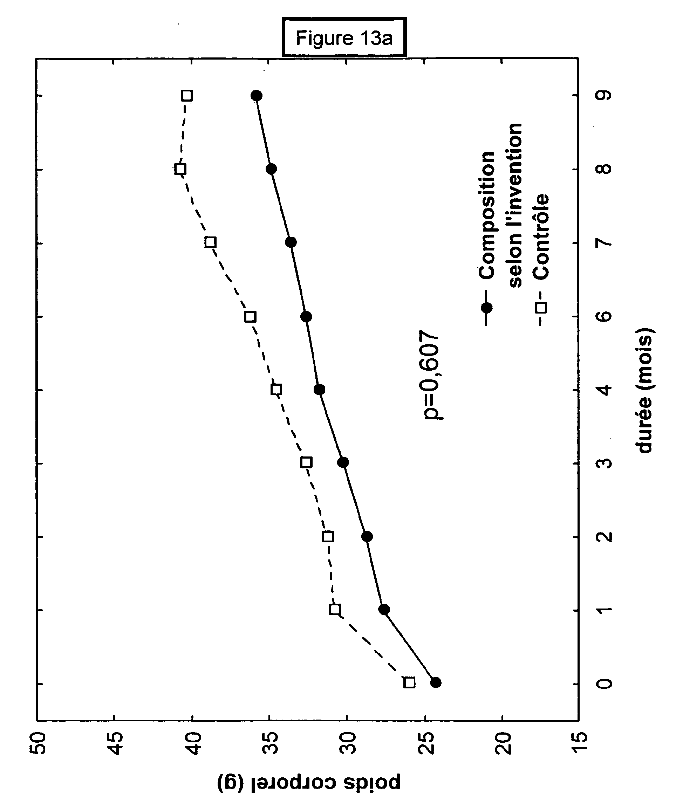

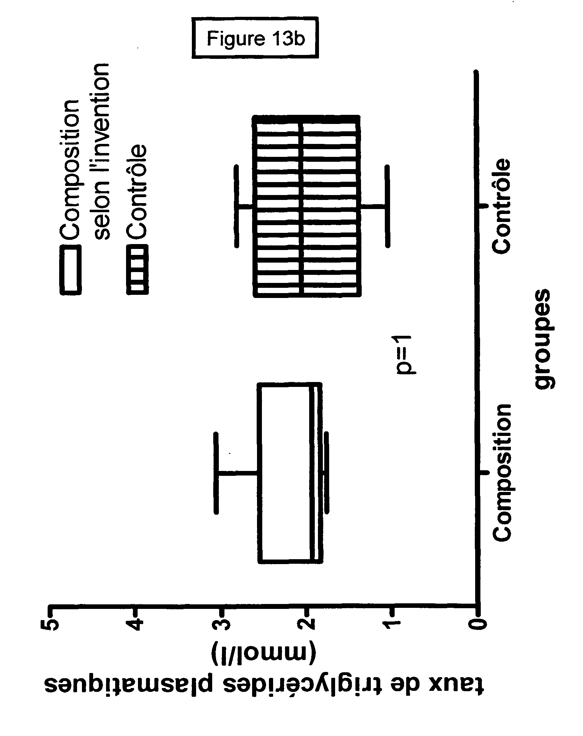

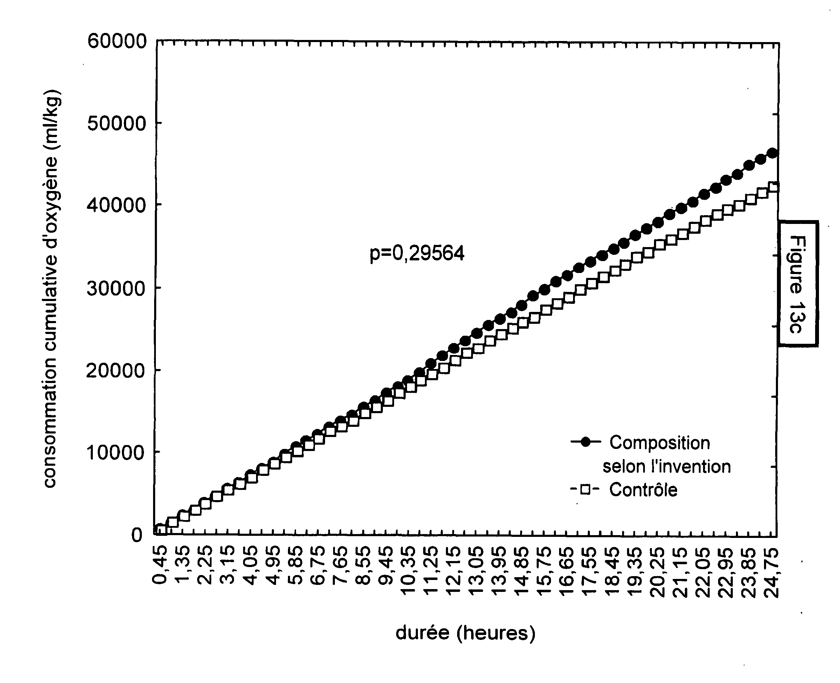

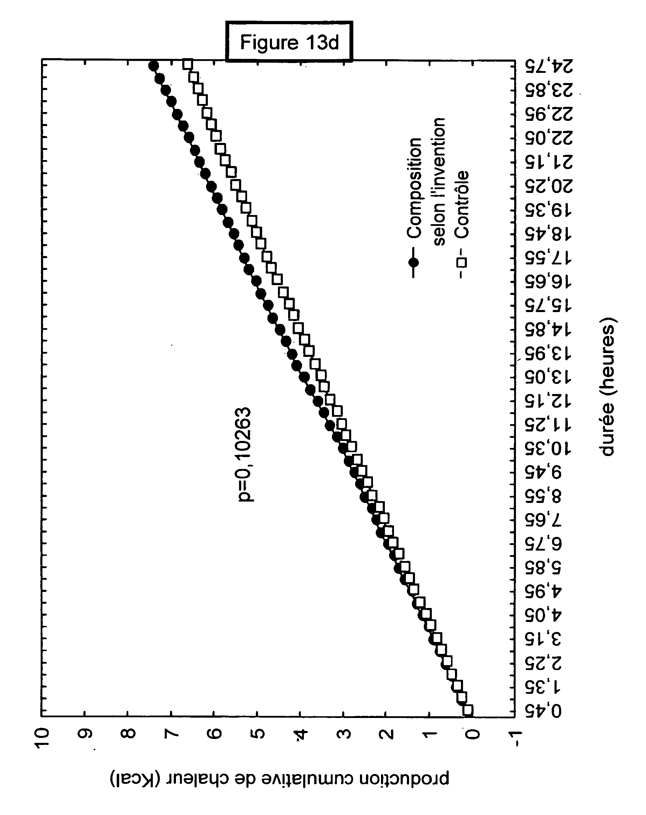

*La* *figure 13* illustre la disparition des effets de la composition selon l'invention chez les souris LDLr ko déficientes en PPARalpha aussi bien sur le poids corporel (figure 13a) que sur le taux de triglycérides plasmatiques (figure 13b) et sur la consommation de d'oxygène (figure 13c) et la production de chaleur (figure 13d).

L'utilisation d'un modèle de souris knock-out (ko), c'est-à-dire déficientes pour un gène donné, est une méthode de choix pour l'étude de la fonction d'un gène et de son importance dans une régulation donnée. Dans le cas précis de la figure 13, l'utilisation du modèle de souris ko pour le gène PPARalpha a permis d'affirmer l'importance de ce récepteur en tant que médiateur des effets métaboliques de la composition selon l'invention.
En effet, la limitation du gain de poids, la stabilisation du taux de triglycérides plasmatiques ainsi que l'augmentation du métabolisme oxydatif (augmentation de la consommation d'oxygène couplée à une augmentation de la production de la chaleur) observés chez des souris mâles LDLrko traités pendant 10 mois avec la composition selon l'invention disparaissent dès que le gène PPARalpha est invalidé chez cette même lignée de souris, rendue doublement déficiente aussi bien pour LDLr que PPARalpha. Ainsi, l'expérience dont les résultats sont représentés par les figures 13a, b, c et d a pour but de vérifier si les effets métaboliques de la composition selon l'invention sont tributaires d'une activation du récepteur PPARalpha.
Deux groupes de souris mâles LDLr ko-PPARalpha ko ont reçu dès l'âge de 2mois, soit la composition selon l'invention incorporée dans la nourriture pour le premier groupe, soit le placebo également incorporé dans la nourriture pour le deuxième groupe. Les deux groupes expérimentaux ont toujours reçu la même quantité de nourriture. Ils ont été régulièrement pesés pendant les 10 mois de traitement, leur sang a été prélevé par ponction rétro-orbitale à l'état nourri et ont séjourné en cages individuelles calorimétriques en fin de traitement. La figure 13 montre qu'il n'existe aucune différence statistiquement significative entre les deux groupes expérimentaux traité et contrôle pour les souris déficientes en PPARalpha, aussi bien sur le plan de l'évolution du poids corporel (figure 13a) que sur le plan de la stabilisation des taux de triglycérides plasmatiques (figure 13b), de la consommation d'oxygène (figure 13c) et de la production de chaleur (figure 13d). Les effets métaboliques précédemment observés chez les souris mâles LDLrko disparaissant chez les souris mâles LDLr ko-PPARalpha ko nous permettent d'affirmer l'importance primordiale de PPARalpha dans l'efficacité de la composition selon l'invention qui se révèle ainsi être un ligand naturel de ce récepteur. PPARalpha étant un acteur majeur du catabolisme oxydatif des lipides, les effets de la composition selon l'invention sur le poids corporel, le taux des triglycérides et le métabolisme oxydatif peuvent ainsi être expliqués via une activation directe de PPARalpha par la composition selon l'invention mise en évidence par l'expérience 14 (décrite ci-après).

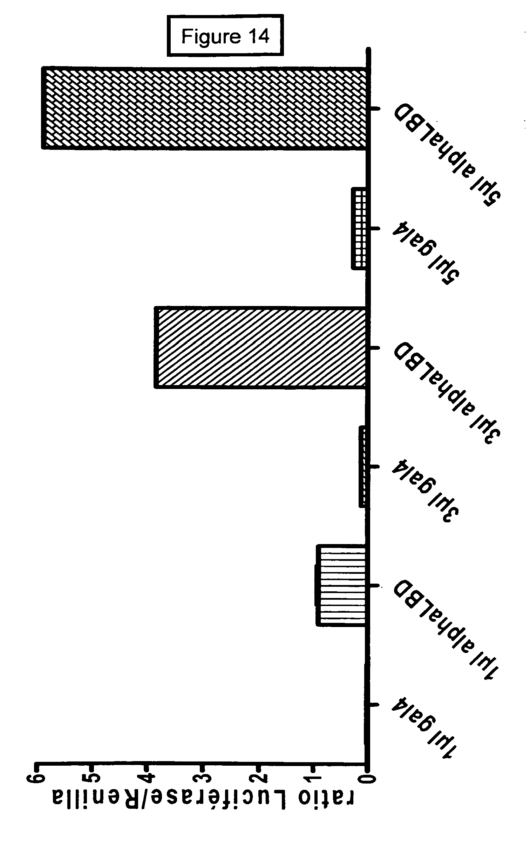

*La* *figure 14* illustre l'activation in cellulo de PPARalpha par la composition selon l'invention avec un effet dose qui confirme cette activation.

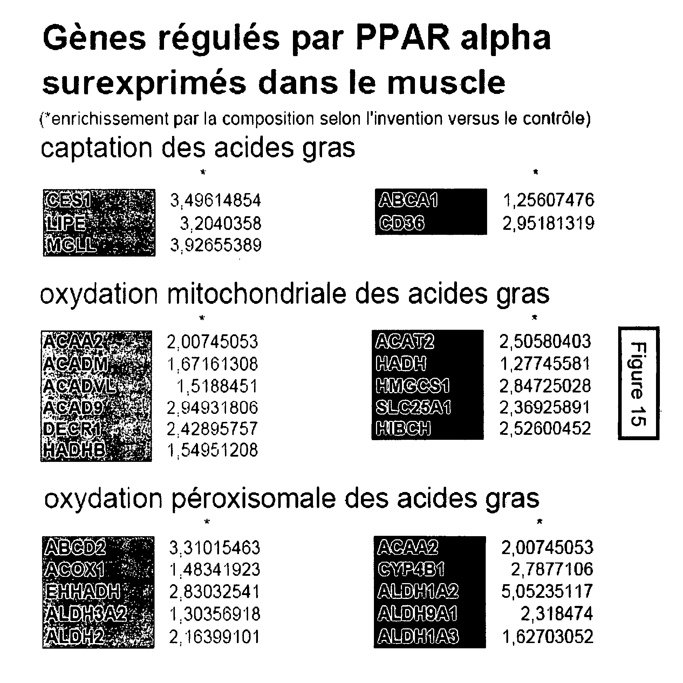

Un test de transactivation est un test qui permet d'affirmer avec certitude qu'une substance donnée active directement un facteur de transcription. PPARalpha est un récepteur nucléaire qui, une fois activé, contrôle la transcription d'un certain nombre gènes.
Afin d'effectuer ce test de transactivation, des cellules NIH3T3 (couramment utilisées pour ce type de test) ont été transfectées par un plasmide contenant un gène de Luciférase sous le contrôle d'un promoteur (5xUAS) qui ne peut être activé que par un facteur de transcription exogène (GAL4) qui est transfecté dans ces mêmes cellules, soit seul, soit lié au domaine de liaison de ligand de PPARalpha (GAL4-alphaLBD). Dans ces mêmes cellules, on introduit également la Rénilla comme contrôle de la transfection et dont le niveau d'expression permet de normaliser celui de la Luciférase.
Ainsi, pour toutes les cultures cellulaires transfectées par Luciférase, Rénilla et GAL4, il n'y a eu presque aucune activation de la transcription de la Luciférase après l'ajout de la composition selon l'invention dans le milieu de culture, alors que celles transfectées par Luciférase, Rénilla et GAL4-alphaLBD ont enregistré une expression de la Luciférase qui augmente proportionnellement à l'augmentation de la dose de la composition selon l'invention dans le milieu de culture. Ainsi, la composition selon l'invention active directement PPARalpha en se liant à son domaine de liaison de ligand et induit ainsi l'expression des gènes cibles de ce récepteur nucléaire et facteur de transcription, comme le montre la figure 15 (décrite ci-après).

*La* *figure 15* illustre l'activation de PPARalpha par la composition selon l'invention qui se traduit par une surexpression de tous les gènes cibles dont la transcription est dépendante de cette activation.

La surexpression des gènes cibles de PPARalpha prouve son activation chez les souris mâles OB/OB traités par la composition selon l'invention pendant 4 semaines versus contrôle.
Deux groupes de souris mâles OB/OB ont reçu dès l'âge de 2mois, soit la composition selon l'invention incorporée dans la nourriture pour le premier groupe, soit le placebo également incorporé dans la nourriture pour le deuxième groupe. Les deux groupes expérimentaux ont toujours reçu la même quantité de nourriture. Après un mois de traitement, les souris ont été disséquées et des échantillons de muscle squelettique ont été prélevés afin d'analyser le profile d'expression génétique induit par la composition selon l'invention dans ce tissu.
Parmi les gènes induits par la composition selon l'invention dans le muscle, tous ceux dont l'expression est dépendante de l'activation de PPAR alpha sont surexprimés **(**Rakhshandehroo M, Sanderson LM, Matilainen M, Stienstra R, Carlberg C, de Groot PJ, Müller M, Kersten S.Comprehensive Analysis of PPARalpha-Dependent Regulation of Hepatic Lipid Metabolism by Expression Profiling. PPAR Res. 2007;2007:26839.). Ces gènes sont tous impliqués dans la captation des acides gras par le muscle et de leur oxydation, aussi bien au niveau mitochondrial que péroxisomal.
Ainsi, la composition selon l'invention induit le catabolisme des lipides et leur utilisation comme substrat énergétique par le muscle. Cette augmentation de l'utilisation des lipides par le muscle est en accord avec l'augmentation de l'endurance musculaire décrite dans l'exemple 8. En effet, lors des exercices d'endurance le muscle utilise de préférence les lipides comme substrat énergétique et réciproquement une augmentation de l'utilisation des lipides par le muscle le rend donc plus endurant. D'autres explications pouvant être apportées à cette augmentation de l'endurance musculaire sont une augmentation de la trophicité et de la contractilité musculaires.

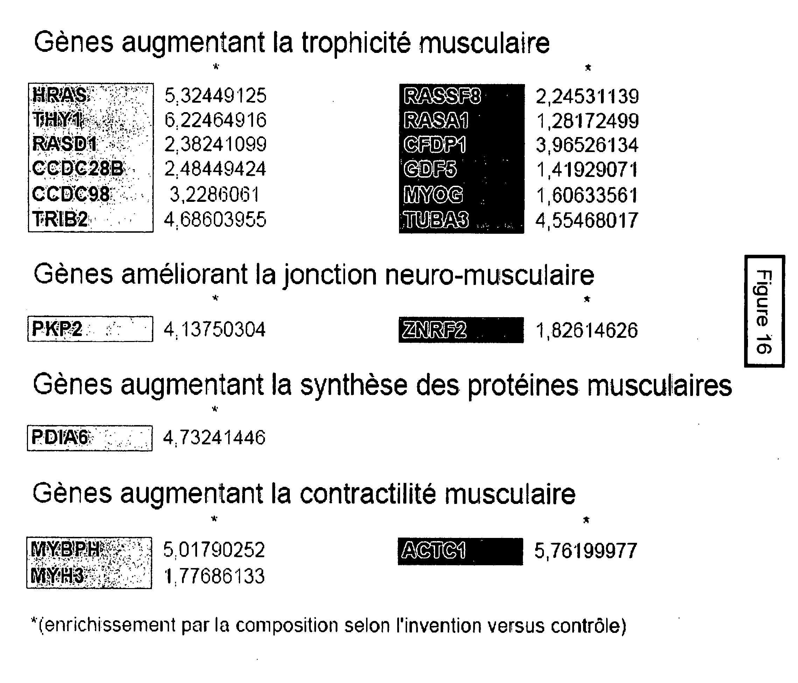

*La* *figure 16* illustre l'augmentation de l'expression de gènes contrôlant la trophicité, la jonction neuro-musculaire et la contractilité musculaire par la composition selon l'invention versus contrôle.

Pour l'expérience dont un des résultats est représenté sur la figure 16, deux groupes de souris mâles OB/OB ont reçu dès l'âge de 2 mois, soit la composition selon l'invention incorporée dans la nourriture pour le premier groupe, soit le placebo également incorporé dans la nourriture pour le deuxième groupe. Les deux groupes expérimentaux ont toujours reçu la même quantité de nourriture. Après un mois de traitement, les souris ont été disséquées et des échantillons de muscle squelettique ont été prélevés afin d'analyser le profile d'expression génétique induit par la composition selon l'invention dans ce tissu.
L'augmentation de l'endurance par la composition selon l'invention (voir exemple 13) peut être expliquée par des fibres musculaires de meilleure qualité grâce à l'enrichissement de l'expression des gènes CCDC28B et CCDC98 qui favorisent la formation de faisceaux musculaires solides qui résisteront ainsi plus longtemps aux microlésions induites par un exercice endurant. Un des gènes les plus essentiels à la trophicité musculaire est HRAS dont l'absence est à l'origine du syndrome dysmorphique de Costello, caractérisé par des tendons fins et un risque de cancer du muscle de 15%. Chez les animaux traités par la composition selon l'invention, l'expression du gène HRAS est plus de 5 fois enrichie versus contrôle.
Le gène PDIA6 dont l'expression est presque 5 fois plus enrichie chez les animaux qui ont reçu la composition selon l'invention versus contrôle participe à la synthèse de nouvelles protéines musculaires qui permettront une réparation efficace et rapide des microlésions sus-citées. Une augmentation de l'endurance implique également une bonne capacité contractile. Ainsi, l'expression de protéines contractiles clés comme ACTC1 et MYBPH est plus de 5 fois enrichie chez les animaux traités par la composition selon l'invention versus contrôle. Une bonne capacité contractile dépendant d'un bon fonctionnement de la plaque de jonction neuro-musculaire, il est tout a fait logique que l'expression des gènes tels que PKP2 et ZNRF2, impliqués dans la transmission de l'influx nerveux au muscle, soit enrichie chez les animaux traités. La composition selon l'invention se positionne donc comme un traitement d'appoint des dystrophies musculaires, et entre autres celles dues à une baisse de la transmission neuromusculaire, ou tout simplement à une immobilisation prolongée (comme lors d'une fracture), en facilitant une récupération rapide du bon fonctionnement **musculaire lors des exercices de rééducation.**

### Exemple 17 : Effets génomiques de la composition selon l'invention

### A. Le muscle est la cible principale de l'action génomique la composition selon l'invention

### Déroulement de l'expérience:

Des souris obèses de type Ob/ob ont été nourries durant 4 semaines avec une diète standard contenant ou non la composition selon l'invention. L'Acide Ribo Nucléique (ARN) contenant l'information relative à l'expression des gènes a été extrait du foie, du muscle gastrocnemius-soleus et du tissu adipeux blanc épididymal. Ces ARNs ont été purifiés, marqués et hybridés sur des puces Affymetrix Mouse Genome 430 2.0.
L'analyse statistique des données a été effectuée en utilisant le langage de programmation statistique R (R Core, 2004, http://www.R-project.org). Les données ont été normalisées pour chaque tissus séparément en utilisant RMA (Irizarry, R. A., et al., 2003), et les gènes régulés par la composition selon l'invention ont été identifiés par modélisation linéaire avec le programme *limma* (Smyth, G. K.. 2004).

### Résultats:

La figure 17A montre que la composition selon l'invention agit principalement sur le muscle. Les gènes régulés ont été sélectionnés de façon à avoir un taux de faux positif inférieur à 10%. 386 gènes sont exprimés de façons différentielles dans le muscle, mais aucun dans le foie ou le tissus adipeux. Toutefois, cette expérience ne permet pas d'exclure d'autres organes cibles non testés. Finalement, cette étude ne peut exclure que la composition selon l'invention induit une modification structurelle de l'ARN, ou une modification de la synthèse ou de la structure des protéines du foie ou du tissu adipeux, ainsi qu'un effet sur l'expression d'un faible nombre de gènes qui auraient pu être manqués en raison du manque de puissance du test statistique.

### B. La composition selon l'invention agit sur des gènes impliqués dans l'activité métabolique du muscle

### Déroulement de l'expérience:

Il a été testé si des gènes appartenant à une voie métabolique ou de signalisation particulière étaient systématiquement sur-exprimés ou sous-exprimés après traitement avec la composition selon l'invention. Pour cela, il a été effectué une analyse dite d'enrichissement de groupes de gènes (« gene set enrichment analysis ») similaire à celle proposée par Mootha et al (2003). Il a toutefois été apporté une modification afin de pouvoir calculer les scores d'enrichissement à partir des valeurs statistiques calculées par le programme *limma*. La signification des scores a été estimée par permutations aléatoires des échantillons pour estimer la distribution nulle des valeurs. Les groupes de gènes représentant les voies cellulaires canoniques proviennent de la base de données « Molecular Signatures Database » (MSigDB, www.broad.mit.edu/gsea/msigdb/).

### Résultats:

L'analyse d'enrichissement de groupes de gènes indique que la composition selon l'invention provoque une altération significative de 26 voies canoniques. Parmi celles-ci, plusieurs voies métaboliques sont affectées : le métabolisme du pyruvate, le cycle de Krebs, la phosphorylation oxydative, les voies du métabolisme des acides gras butanoate, propanoate et pantothenate, ainsi que la biosynthèse de l'acétyl-CoA (Figure 17B). Ceci montre une augmentation générale de la capacité métabolique des cellules musculaires sous traitement avec la composition selon l'invention. Une augmentation de l'activité métabolique des mitochondries va produire un stress oxydatif (« reactive oxygen species », ROS) qui va endommager la cellule. Cependant, chez la souris traitée avec la composition selon l'invention, on observe une augmentation de l'ARN codant pour les superoxides dismutases 2 et 3 qui sont les régulateurs principaux des ROS et qui catalysent leur dégradation. Ces enzymes font partie du « longevity pathway ». Plusieurs voies de signalisation intracellulaires sont aussi affectées positivement par la composition selon l'invention (les voies Biopeptides, Spry, Pyk2, Igf-1, Il-6, Egf, fMLP, p38 et GPCR). Ceci est contraire à ce qui se passe lors du syndrome de résistance à l'insuline où une défectuosité de ces voies de signalisations est observée.

### Références

Irizarry, R. A., et al. (2003). Exploration, Normalization, and Summaries of High Density Oligonucleotide Array Probe Level Data. Biostatistics 4:249-64.
Mootha VK, et al. (2003). PGC-lalpha-responsive genes involved in oxidative phosphorylation are coordinately downregulated in human diabetes. Nat Genet. 34:267-73.
Smyth, G. K.. 2004. Linear models and empirical Bayes methods for assessing differential expression in microarray experiments. Stat. Appl. Genet. Mol. Biol. 3: 3.

### C) Etudes cliniques préliminaires chez l'homme

Les présentes études sont conduites sur une population d'adultes répondant aux critères suivants :
- Homme et femme quelque soit le sexe, l'âge doit être compris entre 18 et 80 ans
- Après échec du régime hygiéno-diététique sur une période de 3 mois
- Personnes présentant un cholestérol total ≥ à 2.30g/l (6.05mmol/l), LDL-C ≥ 1.50g/l (3.9mmol/l) et/ou des triglycérides ≥1.50g/l (1.69mmol/1)
- L'exercice physique et la prise de compléments alimentaires doivent être stables et inchangés pendant la période de l'étude
- La prise d'aliments ayant une action sur la baisse de cholestérol: son d'avoine, protéines de soja supérieure à 10g/j doit être fixe au moins 3 mois avant le début de l'étude et ne doit pas être changée pendant la période de l'étude.

Sont exclues des présentes études les personnes suivantes:
- Porteuses d'une hypercholestérolémie familiale connue et sévère.
- Poursuivant un traitement médicamenteux hypolipémiant : résines, fibrates, statines, acide nicotinique, une période de 3 mois est nécessaire après l'arrêt du produit.
- Prenant des stanols, inhibiteur sélectif de l'absorption intestinale du cholestérol, oméga3
- Femmes enceintes
- Souffrant d'une maladie cancéreuse ou n'étant pas jugées aptes à suivre l'intervention nutritionnelle
- Souffrant d'un diabète 1 (la période de l'étude n'étant pas assez longue pour obtenir la régulation)
- N'ayant pas 18 ans

### Exemple 18 : Métabolisme des lipides en utilisant une composition de l'invention selon l'exemple 1

L'intervention nutritionnelle est une étude randomisée, ouverte sur une période de test de 12 semaines et réalisée sur une population de 104 adultes (45 hommes et 59 femmes) d'âge moyen : 60.7 ans, présentant à J0 un taux moyen de cholestérol de 2.76g/l (7.26mmol/l) et de LDL-C de 1.9g/l (4.94 mmol/l). Cette population d'adultes est répartie en 2 groupes :
- Hypercholestérolémie isolée (75 cas)
- Hyperlipidémie mixte (29 cas)
Au cours de la période étudiée, les patients ne modifient pas leurs habitudes alimentaires antérieures.
Dès le premier jour de l'étude, la composition obtenue selon l'exemple 1 est prise quotidiennement (minimum 2 cuillérées à soupe) à chaque repas, avec les conseils d'utilisation systématique pour toutes les préparations culinaires (assaisonnement et fritures).
Des contrôles sanguins sont effectués à JO puis à 4, 8 et 12 semaines (CT, LDL-C, HDL, TG) ainsi qu'un examen clinique (poids, taille, TA) et un contrôle de la compliance.
Les résultats obtenus sont rassemblés dans le tableau 1 suivant :

**Tableau 1**

| **Baisse moyenne de CT - LDL-C - TG concernant 81% des cas étudiés** | |
|---|---|
| Population globale (104 adultes) | baisse moyenne de CT : 19% |
| | baisse moyenne de LDL-C : 24% |
| Hypercholestérolémie isolée (75 cas) | baisse moyenne de CT : 18,3% |
| | baisse moyenne de LDL-C : 24,8% |
| Hyperlipidémie mixte (29 cas) | baisse moyenne de CT : 19,5% |
| | baisse moyenne de LDL-C : 23,7% |
| | baisse moyenne de TG : 35,5% |

Les 19 % de cas peu ou non répondeurs se révèlent être porteurs de pathologies thyroïdiennes ou diabétiques. Néanmoins la poursuite du suivi au-delà des 12 semaines montre une amélioration progressive des valeurs de CT et de LDL-C dans 30 % des cas.

### Exemple 19 : Métabolisme des lipides en composition de l'invention selon l'exemple 3

Les critères d'inclusion et d'exclusion sont identiques à la précédente étude. L'intervention nutritionnelle est une étude réalisée sur une période de test de 4 semaines sur une population de 35 adultes (13 hommes et 22 femmes) d'âge moyen : 61.1 ans, présentant à JO un taux moyen de cholestérol de 2.85g/l (7.5mmol/l) et de LDL-C de 2.02g/l (5.25 mmol/l). Dès le premier jour de l'étude, la composition obtenue selon l'exemple 3 est prise quotidiennement matin et soir en dehors des repas.
Les contrôles cliniques et biologiques (JO et 4 semaines) sont identiques.
Les résultats obtenus sont regroupés dans le tableau 2 suivant :

**Tableau 2**

| **Baisse moyenne de CT - LDL-C - TG concernant 80% des cas étudiés** | |
|---|---|
| Population globale (35 adultes) | baisse moyenne de CT : 18,5% |
| | baisse moyenne de LDL-C : 24% |
| Hypercholestérolémie isolée (15 cas) | baisse moyenne de CT : 19,6% |
| | baisse moyenne de LDL-C : 24% |
| Hyperlipidémie mixte (13 cas) | baisse moyenne de CT : 17,3% |
| | baisse moyenne de TG : 49% |

Les 20 % de cas peu ou non répondeurs se révèlent être porteurs de pathologies thyroïdiennes, cardio-vasculaires ou diabétiques. Néanmoins la poursuite du suivi au-delà des 4 semaines montre une amélioration progressive des valeurs de CT et de LDL-C dans 34 % des cas.

## Revendications

1. Composition destinée à la régulation du métabolisme des lipides chez l'homme et l'animal **caractérisé en ce que** ladite composition comprend par 100g/100ml la combinaison de:
• 7µg à 700µg d'au moins deux huiles végétales sélectionnées parmi l'huile de colza, l'huile d'olive, l'huile de pépins de raisin et l'huile d'onagre,
• 10µg à 1000µg de minéraux chargés positivement choisis parmi le sodium, le magnésium et le calcium,
• 10µg à 1000µg de métaux choisis parmi le zinc et le fer,
• 7µg à 700µg de levures ou extraits de levure provenant du genre *Saccharomyces cerevisiae,* **caractérisé en ce que** ces dites levures ou extraits de levure sont enrichies en Sélénium,
• 7µg à 700µg de champignons ou d'extraits de champignon Shiitaké (mycelium)
• 6µg à 600µg d'au moins deux extraits végétaux de plante choisis parmi la criste marine, l'ail et la Vigne,
• 8µg à 800µg d'au moins une vitamine choisie parmi les vitamines A, B1, B9, C, E, F et PP
• 7µg à 700µg d'huile animale et de Coprah (Cocos nucifera)
• 6µg à 600µg d'au moins une algue choisie parmi Palmaria palmata (Dulse), Chondrus crispus (Carragaeen) et Fucus vesiculosus (Varech vésiculeux),
et un excipient pharmaceutiquement et/ou alimentairement autorisé.

2. Composition destinée à la régulation du métabolisme des lipides chez l'homme et l'animal selon la revendication 1, **caractérisée en ce que** l'huile animale consiste en de l'huile de poisson des mers froides (Oleum Pisci mare fresca).

3. Composition destinée à la régulation du métabolisme des lipides chez l'homme et l'animal selon la revendication 1 ou 2, caractérisée en ce ladite composition contient au moins deux vitamines choisies parmi les vitamines A, B1, B9, C, E, F et PP

4. Composition selon l'une des revendications précédentes, **caractérisée en ce qu'**elle comprend par 100g/100 ml de :
• 7µg à 700µg d'huile de colza, de l'huile d'olive, de l'huile de pépins de raisin(s), de l'huile d'onagre,
• 10µg à 1000µg de sodium, de magnésium et de calcium,
• 10µg à 1000µg de zinc et le fer,
• 7µg à 700µg de levures ou extraits de levure de *Saccharomyces cerevisiae,* enrichis en Sélénium,
• 7µg à 700µg de mycélium ou d'extrait de mycélium de Shiitake,
• 6µg à 600µg de criste marine, d'ail et de vigne,
• 8µg à 800µg de vitamines A, B1, B9, C, E, F et PP
• 7µg à 700µg d'huile de poisson des mers froides et de Coprah,
• 6µg à 600µg de Palmaria palmata (Dulse), Chondrus crispus (Carragaeen) et Fucus vesiculosus (Varech vésiculeux).

5. Composition selon l'une quelconque des revendications précédentes, **caractérisée en ce qu'**elle comprend en outre des excipients ou additifs, tels que des agents édulcorants, stabilisants, conservateurs, colorants, émulsifiants ou gélifiants, exhausteurs de goût, acidifiants, arômes.

6. Composition selon l'une quelconque des revendications précédentes, **caractérisée en ce qu'**elle se présente sous forme solide, liquide, huile, de gel, filmstrips, pâte, poudre ou gomme.

7. Composition selon l'une quelconque des revendications précédentes, **caractérisée en ce qu'**elle est adaptée à une administration orale.

8. Composition selon l'une quelconque des revendications précédentes, en tant que complément ou additif alimentaire destinée à être incorporable dans tous types de support alimentaires et/ou boissons.

9. Composition selon l'une quelconque des revendications précédentes, en tant que médicament.

10. Composition selon l'une quelconque des revendications 1 à 9, pour son utilisation comme médicament ou comme produit nutritionnel destiné à la régulation du métabolisme des lipides chez l'homme et l'animal.

11. Composition selon la revendication 10, **caractérisée en ce que** la régulation du métabolisme des lipides consiste en l'entretien et/ou la régénération de l'organisme humain ou animal par le rééquilibrage et la redynamisation des fonctions générales dudit métabolisme comprenant :
la stimulation de la consommation des lipides par l'organisme et/ou
la diminution du taux plasmatique du cholestérol et/ou des triglycérides,

12. Composition selon les revendications 10 et 11, pour son utilisation pour le traitement ou la prévention du syndrome métabolique, la formation de plaques d'athérome, la stéatose hépatique et/ou les maladies cardio-vasculaires.

13. Composition selon la revendication 12, **caractérisée en ce que** le syndrome métabolique comprend les maladies liées au contrôle du poids tel que l'obésité, l'amaigrissement ou la stabilisation de la masse graisseuse corporelle.

14. Composition selon la revendication 11, **caractérisée en ce que** le la stimulation de la consommation des lipides par l'organisme favorise l'augmentation du métabolisme oxydatif et de la consommation d'oxygène.

15. Composition selon la revendication 14, **caractérisée en ce que** ladite composition améliore l'endurance.

16. Utilisation non-thérapeutique de la composition selon l'une quelconque des revendications 1 à 8, pour la nutrition animale ou humaine.

17. Composition selon la revendication 10, **caractérisée en ce qu'**elle participe à la baisse du taux plasmatique du cholestérol et/ou des triglycérides.

18. Composition selon la revendication 10, **caractérisée en ce qu'**elle participe à la diminution de l'absorption intestinale des lipides alimentaires.

19. Composition selon la revendication 10, **caractérisée en ce qu'**elle participe à l'augmentation de la motricité musculaire en améliorant la trophicité et la contractilité du muscle.

## Claims

1. Composition adapted to the regulation of the metabolism of lipids in humans and animals **characterized in that** said composition comprises per 100g/100m1 the combination of:
• 7µg to 700µg of at least two plant oils selected among colza oil, olive oil, grape seeds oil, and evening primrose oil,
• 10µg to 1000µg of positively charged minerals selected among sodium, magnesium, and calcium,
• 10µg to 1000µg of metals chosen among zinc and iron,
• 7µg to 700µg of yeast or yeast extracts originated from genius *Saccharomyces cerevisiae,* **characterized in that** said yeasts or yeast extracts are enriched in Selenium,
• 7µg to 700µg of mushrooms or mushroom extracts from Shiitake (mycelium)
• 6µg to 600µg of at least two plant extracts from plants chosen among rock samphire, garlic, and grapevine,
• 8µg to 800µg of at least one vitamin chosen among vitamins A, B1, B9, C, E, F, and PP,
• 7µg to 700µg of animal oil and Copra oil (Cocos nucifera)
• 6µg to 600µg of at least one alga chosen among Palmaria palmata (Dulse), Chondrus crispus (Carrageen), and Fucus vesiculosus (Bladder wrack),
and a pharmaceutically and/or alimentary authorized excipient.

2. Composition adapted to the regulation of the metabolism of lipids in humans and animals according to claim 1, **characterized in that** the animal oil consists of cold seas fishes' oil (Oleum Pisci mare fresca).

3. Composition adapted to the regulation of the metabolism of lipids in humans and animals according to the claim 1 or 2, **characterized in that** said composition contains at least two vitamins chosen among vitamins A, B1, B9, C, E, F, and PP.

4. Composition according to any of the preceding claims, **characterized in that** it comprises per 100g/100 ml:
• 7µg to 700µg of colza oil, olive oil, grape seeds oil, and evening primrose oil,
• 10µg to 1000µg of sodium, magnesium, and calcium,
• 10µg to 1000µg of zinc and iron,
• 7µg to 700µg of yeasts or yeast extracts from *Saccharomyces cerevisiae,* enriched in Selenium
• 7µg to 700µg of mycelium or mycelium extracts from Shiitake,
• 6µg to 600µg of Rock samphire, Garlic, and Grapevine,
• 8µg to 800µg of vitamins A, B1, B9, C, E, F, and PP
• 7µg to 700µg of cold seas fishes' oil and Copra,
• 6µg to 600µg from Palmaria palmata (Dulse), Chondrus crispus (Carrageen), and Fucus vesiculosus (Bladder wrack).

5. Composition according to any of the preceding claims, **characterized in that** it further comprises excipients or additives, such as sweetening agents, stabilizing agents, preserving agents, dyes, emulsifiers or gelling agents, flavor enhancers, acidifiers, and flavors.

6. Composition according to any of the preceding claims, in the form of a solid, liquid, oil, gel, filmstrips, paste, powder, or gum.

7. Composition according to any of the preceding claims, **characterized in that** it is adapted to an oral administration.

8. Composition according to any of the preceding claims, as a complement or a food additive intended to be incorporable in all forms of food matrices and/or drinks.

9. Composition according to any of the preceding claims, as a medicament.

10. Composition according to any of claims 1 to 9, for use as a medicament or as a nutritional product for the regulation of the metabolism of lipids in humans and animals.

11. The composition according to claim 10, **characterized in that** the regulation of the metabolism of lipids consists in the maintenance and/or the regeneration of the human or animal organism by the rebalancing and the reinstigating of the general functions of the aforesaid metabolism including:
stimulation of the consumption of lipids by the organism and/or
reduction in the plasmatic rate of cholesterol and/or triglycerides,

12. The composition according to claims 10 and 11, for use in the treatment or prevention of the metabolic syndrome, the formation of atheromatous plaques, the hepatic steatosis and/or cardiovascular diseases.

13. The composition of claim 12, **characterized in that** the metabolic syndrome comprises diseases related to weight control such as obesity, slimming or stabilization of the body fat mass.

14. The composition of claim 11, **characterized in that** the stimulation of the consumption of lipids by the organism supports the increase in oxidative metabolism and oxygen uptake.

15. The composition of claim 14, **characterized in that** said composition improves the endurance.

16. Non therapeutic use of the composition according to any of claims 1 to 8, for the animal or human nutrition.

17. The composition of claim 10, **characterized in that** it takes part in the decrease of the plasmatic rate of cholesterol and/or triglycerides.

18. The composition of claim 10, **characterized in that** it takes part in decreasing the intestinal absorption of food lipids.

19. The composition of claim 10, **characterized in that** it takes part in increasing muscular motricity by improving muscle trophicity and contractility.

## Patentansprüche

1. Zusammensetzung zur Regulierung des Fettmetabolismus von Menschen und Tieren **dadurch gekennzeichnet, dass** die Zusammensetzung folgende Kombination enthält per 100g/100ml
• 7µg bis 700µg von mindestens zwei Pflanzenölen ausgewählt aus Rapsöl, Olivenöl, Traubenkernöl und Nachtkerzenöl,
• 10µg bis 1000µg positive geladene Mineralien ausgewählt aus Natrium, Magnesium und Calcium,
• 10µg bis 1000µg Metalle ausgewählt aus Zink und Eisen,
• 7µg bis 700µg Hefe oder Hefeextrakte welche aus der Gattung *Saccharomyces cerevisiae* stammt, **dadurch gekennzeichnet, dass** diese Hefe oder dieser Hefeextrakt mit Selen angereichert ist,
• 7µg bis 700µg Pilze oder Pilzextrakte von Shiitake (mycelium),
• 6µg bis 600µg von mindestens zwei Pflanzenextrakten, wobei die Pflanzen ausgewählt sind aus Meerfenchel, Knoblauch und Weinreben,
• 8µg bis 800µg von mindestens einem Vitamin ausgewählt aus A, B1, B9, C, E, F, und PP,
• 7µg bis 700µg Tieröl und Kopraöl (Cocos nucifera),
• 6µg bis 600µg von mindestens einer Alge ausgewählt aus Palmaria palmata (Dulse), Chondrus crispus (Carrageen) und Fucus vesiculosus (Blasentang),
und ein Hilfsstoff, der für pharmazeutische Produkte oder für Lebensmittelprodukte zugelassen ist.

2. Zusammensetzung zur Regulierung des Fettmetabolismus von Menschen und Tieren gemäss Anspruch 1, **dadurch gekennzeichnet, dass** das Tieröl aus Fischöl von Fischen aus Kaltwassermeeren besteht (Oleum Pisci mare fresca).

3. Zusammensetzung zur Regulierung des Fettmetabolismus von Menschen und Tieren gemäss Anspruch 1 oder 2, **dadurch gekennzeichnet, dass** die Zusammensetzung mindestens zwei Vitamine enthält ausgewählt aus A, B1, B9, C, E, F, und PP.

4. Zusammensetzung gemäss einem der vorherigen Ansprüche **dadurch gekennzeichnet, dass** die Zusammensetzung folgende Kombination enthält per 100g/100ml
• 7µg bis 700µg Rapsöl, Olivenöl, Traubenkernöl, Nachtkerzenöl,
• 10µg bis 1000µg Natrium, Magnesium und Calcium,
• 10µg bis 1000µg Zink und Eisen,
• 7µg bis 700µg mit Selen angereicherte Hefe oder Hefeextrakte welche aus der Gattung *Saccharomyces cerevisiae* stammt,
• 7µg bis 700µg Pilze oder Pilzextrakte von Shiitake,
• 6µg bis 600µg Meerfenchel, Knoblauch und Weinreben,
• 8µg bis 800µg Vitamine A, B1, B9, C, E, F, und PP,
• 7µg bis 700µg Fischöl von Fischen aus Kaltwassermeeren und Kopraöl (Cocos nucifera)
• 6µg bis 600µg Palmaria palmata (Dulse), Chondrus crispus (Carrageen), und Fucus vesiculosus (Blasentang).

5. Zusammensetzung gemäss einem der vorherigen Ansprüche **dadurch gekennzeichnet, dass** die Zusammensetzung ausserdem Hilfsstoffe und Additive enthält wie Süssungsmittel, Stabilisierungsmittel, Konservierungsmittel, Farbstoffe, Emulgatoren oder Geliermittel, Geschmacksverstärker, Säuerungsmittel, Aromastoffe.

6. Zusammensetzung gemäss einem der vorherigen Ansprüche als Feststoff, Flüssigkeit, Öl, Gel, Filmstreifen, Paste, Puder oder Gummi.

7. Zusammensetzung gemäss einem der vorherigen Ansprüche **dadurch gekennzeichnet, dass** die Zusammensetzung zur oralen Verabreichung geeignet ist.

8. Zusammensetzung gemäss einem der vorherigen Ansprüche als Ergänzungsmittel oder Nahrungszusatz zur Einarbeitung in alle Formen von Lebensmitteln und/oder Getränken geeignet.

9. Zusammensetzung gemäss einem der vorherigen Ansprüche als Medikament.

10. Zusammensetzung gemäss einem der Ansprüche 1 bis 9 zur Anwendung als Medikament oder als Nahrungsprodukt zur Regulierung des Fettmetabolismus von Menschen und Tieren.

11. Die Zusammensetzung gemäss Anspruch 10 **dadurch gekennzeichnet, dass** die Regulierung des Fettabbaus in der Aufrechterhaltung und/oder in der Regeneration des menschlichen oder tierischen Organismus besteht durch wieder Ausgleich und wieder Anregung der allgemeinen Funktionen des oben erwähnten Metabolismus umfassend die Stimulierung des Fettverbrauchs durch den Organismus und/oder die Reduktion des plasmatischen Anteils von Cholesterol und/oder von Triglyceriden.

12. Die Zusammensetzung gemäss Anspruch 10 und 11 zur Anwendung in der Behandlung oder Vorbeugung des metabolischen Syndroms, der Bildung von atheromatischen Ablagerungen, Fettleber und/oder Herz-Kreislauferkrankungen.

13. Die Zusammensetzung gemäss Anspruch 12 **dadurch gekennzeichnet, dass** das metabolische Syndrom Krankheiten die Gewichtskontrolle betreffend umfasst wie Fettleibigkeit, der Abbau oder die Stabilisierung der Körperfettmasse.

14. Die Zusammensetzung gemäss Anspruch 11 **dadurch gekennzeichnet, dass** die Stimulierung des Fettverbrauchs durch den Organismus die Erhöhung des oxidativen Metabolismus und die Sauerstoffaufnahme fördert.

15. Die Zusammensetzung gemäss Anspruch 14 **dadurch gekennzeichnet, dass** die Zusammensetzung die Ausdauer verbessert.

16. Nicht therapeutische Verwendung der Zusammensetzung gemäss einem der Ansprüche 1 bis 8 zur Ernährung von Tieren und Menschen.

17. Die Zusammensetzung gemäss Anspruch 10 **dadurch gekennzeichnet, dass** die Zusammensetzung an der Reduktion des plasmatischen Anteils von Cholesterol und/oder von Triglyceriden beteiligt ist.

18. Die Zusammensetzung gemäss Anspruch 10 **dadurch gekennzeichnet, dass** die Zusammensetzung die Absorption von Nahrungsfetten im Darm erniedrigt.

19. Die Zusammensetzung gemäss Anspruch 10 **dadurch gekennzeichnet, dass** die Zusammensetzung die Bewegungsfähigkeit der Muskeln erhöht durch Verbesserung der Muskeltrophik und der Muskelkontraktilität.
